# EUROPEAN PATENT SPECIFICATION

(11) **EP 2 004 057 B1**
(45) Date of publication and mention of the grant of the patent: **24.11.2010**
(21) Application number: 07712891.6
(22) Date of filing: 13.03.2007
(51) Int. Cl.: A61B 5/151

(54) **METHOD AND APPARATUS FOR PIERCING THE SKIN AND DELIVERY OR COLLECTION OF LIQUIDS**
VERFAHREN UND GERÄT ZUR DURCHSTECHUNG DER HAUT UND ABGABE ODER SAMMLUNG VON FLÜSSIGKEITEN
MÉTHODE ET APPAREIL POUR PERCER LA PEAU, ET ADMINISTRATION OU PRÉLÈVEMENT DE LIQUIDES

(30) Priority: 13.03.2006 GB 0605003
(43) Date of publication of application: 24.12.2008
(73) Proprietor: Microsample Ltd., Thicket Road, Houghton Huntingdon Cambridgeshire PE28 2BQ (GB)
(72) Inventor: CASSELLS, John, Maclaren, Huntingdon Cambridgeshire PE28 2BQ (GB); HARMAN, Anthony, David, Rotherfield Peppard Oxfordshire RG9 5JP (GB)
(74) Representative: Naylor, Matthew John
(86) International application number: PCT/GB2007/000861
(87) International publication number: WO 2007/104960

(56) References cited:
- WO-A-2005/094680
- US-A- 5 569 287
- US-A1- 2004 034 318
- US-B1- 6 540 762

## Description

### BACKGROUND TO THE INVENTION

### Field of the invention

This invention relates to a method and apparatus for making an incision or puncture into the skin, or for delivering a liquid to the skin and making an incision or puncture through said liquid, or for making an incision or puncture into the skin and collecting liquid from the surface of the skin, and transferring said liquid to another device or receptacle. The process of making an incision or puncture into the skin is to create a wound to cause blood or interstitial fluid to be evolved as a prerequisite to sampling said blood or interstitial fluid. The process of delivering a liquid to the skin, followed by piercing the skin through the liquid, is used to elicit an allergic response (for example, the skin prick test for diagnosing allergies), and for vaccination (for example, against smallpox). The process of making an incision in the skin followed by aspiration of blood or interstitial fluid (ISF) resulting from the incision and transferring said blood or fluid to another device or receptacle is used in Point-of-Care (POC) and other patient testing.

### Related art

Lancets and lancing devices for lancing the skin to create a wound to evolve blood are well known in the art. WO97/46157 describes a common type of lancing device for use with a disposable lancet. A tip on the lancing device is removed to allow insertion of a lancet. The tip is then replaced, enclosing the lancet. In WO97/46157 the tip is adjustable to control the depth of penetration of the lancet in use. Lancing devices of this common configuration often have additional features, such as adjustable spring force, to further control the action of the lancet. A related art is that of safety lancets, WO2004/039429 featuring a typical example. Safety lancets are designed to be used once and to automatically retract the lancet within a lancet housing so that there is a reduced chance of needlestick injury. Safety lancet devices incorporate a spring-loaded lancing mechanism. They are intended to be disposable in their entirety, and therefore for reasons of cost and size do not incorporate precision mechanisms to adjust depth of penetration and force.

US2004/0034318 discloses a system for piercing the skin. The system comprises a lancet unit and a drive unit. The lancet unit has a lancet with a sharp tip. The drive unit is capable of releasably engaging with the lancet unit.

The most commonly used test for diagnosing allergies is the skin prick test. This is performed by dispensing a drop of liquid containing a selected allergen to the skin surface and lancing the skin through the dispensed drop of liquid. It is not necessary to lance the skin deeply for this test, as the intent is to damage the skin and provoke a histamine-mediated inflammation reaction, rather than produce a sample of blood at the puncture site. A closely related test is the scratch test where the skin is scratched rather than lanced, often with a multi-faceted or multi-tined device.

The most commonly used devices for performing the skin prick test are the dropping pipette and hand-held lancet. Allergen solutions are supplied in small vials containing typically 2-3 ml of solution. A dropping pipette consisting of a small glass pipette with rubber bulb is included with the vial, often as part of the cap. The physician uses the pipette to deposit a droplet of liquid onto the patient's skin. The volume of this droplet is not accurately controlled and is typically around 40 µl. The physician pierces through the droplet using a hand-held lancet with a small sharp point, typically 1- 1.2mm long. The lancets may be made entirely of metal (for example LETI SA prick test lancets) or may comprise an insert-moulded steel lancet, or may be made entirely of plastic.

The Morrow Brown needle is an all-plastic device with a relatively sharp point. Just before use, the tip of the device is immersed in an open well of allergen. When the device is removed from the well a droplet of allergen adheres to the device and is transferred to the skin at the puncture site just as the lancet pierces the skin. It has been reported that plastic needles or lancets used for allergy testing are more painful than metal needles, probably because the plastic point is an injection moulded feature rather than a fine sharp cutting point formed by grinding, as in metal lancets.

Other commercial lancet devices are available for delivering allergens to the skin before piercing or scratching the skin in a single operation. Examples include the insert moulded Quintip^{™} from Hollister Steer Laboratories LLP, and the all-plastic GreerPick^{™} from Greer Laboratories. These devices are first dipped into a well of allergen solution and employ surface tension to pick up a droplet of allergen solution from an open reservoir for transfer to the skin and a fixed integral lancet or pick to pierce or scratch the skin. US5647371 describes an extension of the basic GreerPick device where an array of picks is immersed in wells of allergen before being applied to the skin as a unit to perform a number of tests simultaneously. US5944671 describes a handle for holding several pick devices, the object being to reduce the amount of waste plastic by having disposable picks and a re-usable handle. US6095988 offers an alternative applicator handle design for such multi-testing, with improved ergonomics and reduced cross-contamination between adjacent tests

Various other innovations have been proposed for allergy testing, including a variation on the GreerPick comprising a central recessed feature to limit skin penetration of the tines more controllably, whilst still allowing capillary pickup of the allergen solution. (US5820562), a vial of allergen with a stopper incorporating a scratching device (EP0292928), adhesive strips incorporating allergens and lancets (EP0081975A2, US4802493, US4966159, US5099857) and a device to pick up allergen solution from a well by capillary action and transfer it to the skin within a hollow cannula (US4270548).

The method of making an incision through a liquid and devices for performing the same can also be used for some vaccinations, for providing a therapeutic immune response, or to deliver a therapeutic drug or other substance in the form of a colloid, suspension or solution. Vaccination with smallpox by scarification is known from ancient times, and the use of cowpox for vaccination against smallpox was performed by this process in 1796 by Edward Jenner. Several devices have been used to administer smallpox vaccine by penetration of the stratum corneum to the deep epidermis, including scalpel-like devices, the rotary lancet and straight needles. Benjamin Rubin developed the bifurcated needle in 1965 that is still used today. This has two tines close together. When dipped into a solution of vaccine and removed approximately 2.5µl of vaccine solution is held between the times by capillary force. Some of this liquid is transferred to the surface of the skin when the needle is used to break the surface of the skin. Smallpox has been almost eradicated in the global population, however there remains interest in simple devices for vaccination in the event of a Smallpox bioterrorism threat and potentially for vaccination against other pathogens, particularly in developing countries.

The prior art discloses several arrangements involving the use of microneedles to deliver liquids beneath the skin by a minimally invasive method. Examples of microneedle devices and arrays for liquid delivery can be found in US2005/143713, US2005/137525, WO2005/049107, WO2003/022330, and CN1562402. Such microneedle systems are designed to deliver a small defined volume of liquid beneath the skin through an incision, not to deliver a liquid to the skin surface before incision through that liquid. US644782 describes an injector to deliver a defined volume of liquid beneath the skin.

Efficient liquid sampling and depositing systems in the form of laboratory pipetting systems are known. US5413006 and EP0364621 represent typical examples of air-displacement pipettors with separate actuator and pipette tip. EP0078724 describes a hand-held positive displacement pipettor with disposable tips. EP1212138 describes a miniaturised positive-displacement pipette capable of aspirating and dispensing sub-microlitre volumes of liquid, while WO0112330 describes how such pipettes may be attached to a continuous strip for automated pipetting. These devices can aspirate and dispense liquids to high accuracy, but have no capability to pierce the skin or to effect an analysis.

The risk of needlestick injury with allergy test devices, bifurcated needles and blood-sampling lancets has become of great concern to healthcare workers and legislators (for example the Needlestick Safety and Prevention Act in the US). Whereas modern medical technology demands ever-higher precision from sampling devices, particularly for quantitative tests, there is also a continual drive to reduce the cost and waste materials from disposable medical devices. It is also recognised that repeatedly opening a reservoir of liquid (for example, allergen solution) that was previously sterile or was filled aseptically, or decanting such solution into open wells for multiple use can lead to airborne contamination of the liquid.

In POC testing, the POC instrument is often a bench-top instrument and cannot be taken to the patient, for example, to take a sample of blood from a patient. In such circumstances it is necessary to take a sample of blood or ISF and transfer such to the instrument. It is further desirable that either the sample itself or the dispensed aliquot of the sample be of an accurately measured volume to ensure accuracy of the resulting test.

The prior art in blood sampling describes devices for combining lancing and liquid sampling. US4360016 discloses a capillary channel adjacent and parallel to a lancet. After withdrawing the lancet from the skin, a droplet of blood may fill the capillary channel by capillary action. WO2004/066822 describes the combination of a lancet and blood glucose test strip with a capillary channel adjacent the tip of the lancet. WO2004/066822 improves upon US4360016 by delivering blood directly to a glucose test strip by capillary action.

WO02/100254 discloses another approach, where a capillary channel is provided with an entrance adjacent a housing containing a lancet, the capillary channel being arranged at an angle to the lancet. A droplet of blood in proximity to the lancet can be drawn by capillary action into the capillary. WO02/056751 describes a lancet within a housing that forms an annular capillary channel concentrically around the lancet between the lancet and the housing. WO2004/060163 also describes a lancet within a capillary tube member such that the clearance between the lancet and housing forms an annular capillary.

Yet another method of capillary sampling is to provide a hollow microneedle in connection with a capillary such that the whole assembly forms a capillary sampling conduit. This may further be integrated with blood glucose sensing. Examples in the prior art include US2004/0096959 and US2003/0153900.

US2003/0088191 describes a lancet attached to a diaphragm. Air pressure behind the diaphragm is used to drive the lancet tip out of an orifice to pierce the skin. Applying a partial vacuum behind the diaphragm serves to withdraw the lancet and create a negative pressure in a chamber to draw in a liquid sample.

US5569287 discloses a blood-sampling device utilising a trigger mechanism to push a needle into a puncture position by operation of a piston. Sampling of a blood drop is achieved by withdrawing the piston along its barrel, creating a partial vacuum in a headspace above the blood drop, drawing a blood sample into a tube surrounding the needle.

Another group of prior art devices are the devices that employ auction to draw blood to the surface of an incision. The purpose of these is to help to draw blood to the surface at "alternative testing sites", such as the arms or legs, that are less painful than the fingers, but are more difficult to draw blood from. US4653513 describes a system having a lancet attached to a plunger.

US5368047 describes an improvement over US4653513 containing a separate lancet and plunger. The inventor describes a disadvantage of US4653513, where the friction of the piston seal in the device is detrimental to the lancing action. US5368047 solves this problem by providing a low friction lancet that does not seal at any point in the bore, and a separate syringe assembly at the other end of the device to creats a vacuum. Three integral springs are used to drive the device. Further examples of this type of device are provided in US2002/111565 and WO9955232.

### SUMMARY OF THE INVENTION

In view of the above discussion, the present inventors therefore consider that it is thus desirable to be able to perform any or a combination of some or all of the actions of liquid delivery, piercing, sample aspiration and sample transfer using a single disposable device of small or minimal size (and waste material), combined with low or minimum cost and complexity in combination with a re-useble actuator. It is further desirable that such a disposable device should incorporate robust needlestick prevention measures, that it maintains the sterility of the lancet until the last possible moment before use, that it is capable of being filled with a sterile or aseptic solution that is maintained sterile or aseptic until the point of use and that it prevents further contamination of a blood or ISF sample as far as possible while transporting said sample. It is also desirable that the lancing and liquid aspirating/dispensing disposable device be engaged and disengaged from the re-usable actuator quickly and easily with a single-handed action.

It is an object of the present invention to address, avoid or even overcome one or more of the problems identified above. For the avoidance of doubt, the problems set out under the heading "Related art" above are not necessarily themselves part of the prior art, but the discussion of those problems identified by the present inventors is included in that section of this specification to assist the reader in understanding the present invention.

Accordingly, in a first aspect, the present invention provides a kit as set out in claim 1.

In another aspect, the present invention provides a method as set out in claim 9.

The present invention allows the use of a disposable single use medical device in order to prick the skin of a subject, e.g. for allergy testing or for causing a wound for blood sampling. It is possible, of course, that such a device could be used more than once, but for safety and hygiene reasons it is preferably disposed of after a single use. The movement of the sharp tip of the lancet piston to a shielded after-use configuration allows for the safe removal of the disposable medical device from an actuator device (without risk of needle stick injury), and for the same actuator device to be re-used with a fresh disposable medical device.

Preferably, the activation means is also operable to cause release of the engagement between the lancet piston and the actuator member at a second axial position of the lancet piston, the sharp tip of the lancet piston being shielded by the lancet housing in said second position. In this way, the same activation means can operate to cause both engagement and disengagement of the actuator member and the lancet piston. This provides for a simplified structure. Preferably, the second axial position is substantially the same position as the first axial position, but these positions need not be identical. However, it is preferred that the sharp tip of the lancet piston is shielded by the lancet housing in the second position.

Furthermore, the position of the lancet piston in the lancet housing in the before-use configuration may be substantially the same as the first axial position. Additionally or alternatively, the position of the lancet piston in the lancet housing in the after-use configuration may be substantially the same as the second axial position.

As indicated above, the activation means is formed as part of the actuator device. In this case, it is preferred that operation of the actuator device to move the actuator member can be carried out at the same time as operation of the activation means to cause releasable engagement of the actuator member with the lancet piston.

The activation means may be formed integrally with a component other than the lancet piston and the actuator member, for example, the lancet housing of the medical device. This allows the activation means, for example, to be a non-moving part, and so allows simplification of the structure. The activation means may, for example, have a surface directed inwardly into said internal space of the lancet housing. Preferably, the activation means is an inwardly-facing abutment surface of said lancet housing, operable so that abutment of a lancet piston engagement means during an operation of the actuator member to move the lancet piston axially causes said releasable engagement between the lancet piston and the actuator member. Most preferably, the inwardly-facing abutment surface of the activation means that co-operates with the lancet piston engagement means is an inwardly-tapering surface. It is preferred that the tapering is an inward tapering in the forward direction of the lancet housing.

More than one activation means may be provided. For example, an activation means formed integrally with the actuator may also act cooperatively with activation means on the lancet housing.

Preferably, an activation means formed integrally with the lancet housing acts as an interlock to prevent release of the engagement between the lancet piston and actuator member at any position forward of the before-use and/or after-use location of the lancet piston.

The clutch jaws are radially deformed by abutment with said activation means during engagement of the lancet piston and the actuator member, said radial deformation operating to retain the actuator member and said lancet piston engaged together in both compression and tension during forward and rearward motion of the engaged assembly.

The clutch jaws may, in operation, engage with a radial step or annular groove or other upset feature formed on said lancet piston or said actuator member to allow the actuator member to pull the lancet piston back towards the after-use configuration from a puncture position. Preferably the engagement is an interlocked engagement so that release of engagement is not possible until the after-use position has been reached.

Preferably, the clutch jaws are resilient. This allows the device to operate so that removal of the clutch jaws from abutment with the activation means allows the reversal of the radial deformation to release the engagement between the lancet piston and the actuator member.

Preferably, there are two, three or more clutch jaws. These may be evenly angularly disposed around the axis of the lancet housing, when the device is assembled with the actuator device, in order that the engagement between the lancet piston and the actuator member is evenly distributed.

The device may include at least one stop element for urging against accidental exposure of the sharp tip of the lancet piston from the lancet housing before or after the lancet piston engagement means is engaged with the actuator member.

The device may include at least one stop element for limiting the projection of the lancet piston from the lancet housing before or after the lancet piston engagement means is engaged with the actuator member.

Preferably, there is provided a co-operating means between the lancet piston and lancet housing to hold the lancet piston in a fixed axial position relative to the lancet housing, such co-operating means being capable of being overcome by operation of the actuator. The co-operating means may consist of a bump-off feature or interference fit between the lancet piston or a component integral with the lancet piston and the lancet housing. In this way the lancet piston is held in a before-use and after-use position when not bering driven by the actuator and with the sharp tip of the lancet piston shielded by the lancet housing, whether or not the disposable medical device is fitted to the actuator.

The device may include at least one locator lancet piston guide element for maintaining the axial and/or radial position of the lancet piston with respect to the lancet housing before and/or after engagement with the actuator member.

Preferably, the lancet housing has a rearward end at which the actuator device attaches, the rearward end having a first inner diameter, the inner diameter of the lancet housing reducing in stages from said rearward end to an intermediate portion of smaller diameter to a bore of smaller diameter still. The lancet piston may slidably and sealingly fit in at least one section of the bore, there being a liquid-containing space in the bore forwardly of the lancet piston in either the before-use configuration or in the after-use configuration or both.

When there is a locator lancet piston guide element present, it is preferably dimensioned to abut against the internal surface of the lancet housing at the intermediate portion in order to maintain the radial position of the lancet piston centrally within the bore.

In this way, the lancet piston guide element allows the maintenance of the radial position of the lancet piston, which can be important for ensuring suitable engagement between the lancet piston and the actuator member.

Preferably, the device has seal means operable substantially to prevent flow of liquid from the liquid-containing space and to prevent air flow into the liquid-containing space past the seal means on movement of the lancet piston or liquid from the liquid-containing space past the seal means on movement of the lancet piston, the seal means being in eliding engagement with a sealing surface, one of the seal means and the sealing surface being fixedly movable with the lancet piston at least during forward displacement of the lancet piston, so that displacement of the lancet piston either from the before-use configuration to the exposed position or from the exposed position to the after-use configuration provides at least one of:
(i) suction for drawing liquid into and along the liquid-containing space from a forward end of the bore, and
(ii) pressure for expelling liquid from the liquid-containing space via a forward end of the bore.

Preferably, the locator lancet piston guide element is releasably retained in a preferred axial position with respect to the lancet housing in the beforo-use configuration. Preferably, the locator lancet piston guide element also maintains the radial position of the lancet piston in both the before-use and the after-use configuration.

The aspects of the invention set out above allow a particular use in which the disposable device is pre-filled with a liquid. In this context, "liquid" includes any flowable condensed matter that includes a liquid component, such as blood, ISF, aqueous solutions, non-aqueous solutions, liquids, liquid mixtures, cell suspensions, or other suspensions of biological material. Where the disposable device is pro-filled with liquid, and it is necessary to dispense some or all of the liquid at a dispensing site, it is strongly preferred that the attachment of the disposable device to the actuator device does not cause any accidental expulsion of liquid from the device. In order to ensure this, it is preferred that the lancet piston does not move forwardly during the attachment operation. Indeed, it is preferred that the lancet piston does not move forwardly or rearwardly during the attachment operation. Such an advantage is provided by ensuring that the lancet piston is held in the bore, e.g. via friction, which can be achieved by suitable choice of relative sizes and shapes for the bore and lancet piston (including any seal). Similarly, to ensure against accidental expulsion of pro-filled liquid from the device, it is preferred that the lancet piston does not move forwardly or rearwardly during engagement of the lancet piston with the actuator member, unless such forward movement is also part of an intentional operation to expel liquid, in which case the engagement operation occurs at or just forward of the before-use or after-use position of the lancet piston in the lancet housing. Such an advantage may be achieved as set out elsewhere in the specification with respect to the engagement of the lancet piston with the actuator member, and the activation means to assist with that engagement.

Further preferred and/or optional features arc now set out. These are applicable either singly or in any combination to any of the aspects of the invention, unless the context demands otherwise. The engagement mechanism is provided at said actuator device. This allows the disposable device to have a simpler construction, the more complex construction of the actuator device being justified because it is intended to be used more than once.

Preferably, the method of operating the kit further includes the steps of:
releasing engagement between the lancet piston and actuator member, optionally via said activation means at the after-use position; and
removing or ejecting the disposable medical device from the actuator, so that the lancet piston tip remains shielded by the lancet housing, e.g. in its after-use position.

Preferably, the lancet housing of the disposable device has a bore within which the lancet piston slidably and/or sealably fits, there being a liquid-containing space in the bore forwardly of the lancet piston in the beforo-use configuration and/or in the after-use configuration.

Preferably, the disposable device has seal means operable substantially to prevent flow of fluid (for example air or liquid) from into or out of the liquid-containing space past the seal means on movement of the lancet piston, the seal means being in sliding engagement with a sealing surface, one of the seal means and the sealing surface being fixedly movable with the lancet piston at least during forward displacement of the lancet piston, so that displacement of the lancet piston either from the before-use configuration to the exposed position or from the exposed position to the after-use configuration provides at least one of:
(i) suction for drawing liquid into and along the liquid-containing space from a forward end of the bore, and
(ii) pressure for expelling liquid from the liquid-containing space via a forward end of the bore.

A liquid may be expelled from the forward end of the bore during forward displacement of the lance piston in order to dispense said liquid at the skin of said subject, prior to lancing. Additionally or alternatively, lancing may cause at least a droplet of blood to form at the surface of the skin, subsequent retraction of the lancet piston along the bore aspirating a sample of blood from the skin surface into said liquid-containing space.

Still further preferred and/or optional features of the invention, and further aspects of the invention, will now be set out. These may be applied singly or in any combination with any aspect (including any preferred or optional features) set out above.

Preferably, the lancet piston acts as a piston within the bore, so that liquid can be moved within the device by positive displacement. The seal may be a piston seal or a rod seal or may be an interference fit.

In use, the lancet piston is projected beyond the end of the bore to penetrate or prick the skin to cause a wound and is then withdrawn. In the case where the lancet piston is used to take a sample from a subject, it is necessary to wait for blood or ISF (interstitial fluid) or other liquid to evolve from the wound, a blood or ISF or other sample can then be drawn into the bore by retraction of the lancet piston where it may be metered and held for subsequent ejection into another device or compartment, or may be transferred to sensors or a chamber for analysis within the bore itself or connecting channels. The device may also deliver liquids by positive displacement to the skin before lancing. The actuator driving the device may be driven manually, by spring, hydraulic, pneumatic, electrical force, or with the aid of a motorised, automated system. Typically, the device is driven by an actuator as set out in further detail below.

The invention provides for a smaller cross-sectional area for the penetrating component than an equivalent hypodermic syringe needle of the same bore as the bore of the device. For example, a hypodermic syringe microneedle with a bore of 430 micrometres diameter would have an outside diameter of perhaps 650 micrometres. With embodiments of the present invention, the lancet piston may have a similar outside diameter (about 430 micrometres) as the bore.

When used to sample liquids, the embodiment of the invention incorporating a lancet piston offers an instant improvement over the use of capillaries for sampling liquids because, by using positive displacement, it may achieve a theoretical head of 10 m of water for all suitable bore diameters compared with a head of only tens of centimetres for practical capillaries of 50 micrometers diameter and above. Some embodiments of the invention are also able to maintain sample flow when the liquid encounters an increase in the diameter of the sampling channel (for example a chamber, step or taper) where capillary flow would otherwise stop.

The use of the positive displacement principle also allows samples of viscous liquids, including those with high solids content, to be aspirated and dispensed without causing separation of the solids content from the bulk liquid. Particulates in a sample are far less likely to block the bore of the device when compared with capillary devices. High solids content liquids include blood with high cell content, partially clotted blood and cell suspensions.

Still further preferred and/or optional features of the invention will now be set out. These are applicable either singly or in any combination with any aspect of the invention, unless the context demands otherwise.

Preferably, the liquid is one of a liquid, a mixture of liquids and a mixture of liquid or liquids with solid or solids.

Preferably, the seal means is in slidable engagement with a sealing surface, one of the seal means and the sealing surface being movable (preferably fixedly movable) with the lancet piston, at least during forward displacement of the lancet piston, Said one of the seal means and the sealing surface may be fixedly movable with the lancet piston during rearward displacement of the lancet piston.

The seal means may be formed by the outer surface of the lancet piston, the sealing surface being an internal surface of the bore. The outer surface of the lancet piston may be profiled to provide the seal means as a surrounding projection. For example, the outer surface of the lancet piston may provide an annular projection. Alternatively, the seal moans may be a sealing member disposed around the lancet piston, the sealing surface being an internal surface of the bore. In these embodiments, the seal means may still move with the lancet piston.

In alternative embodiments, the seal means is a sealing member disposed at the internal surface of the bore, the sealing surface being an outer surface of the lancet piston. Here, the seal means will not usually move with the lancet piston.

Preferably, the seal means is disposed adjacent the lancet piston tip, at least when the lancet piston is in the retracted position. The advantage of this is that it is then possible to avoid a large headspace of compressible gas above the (normally substantially incompressible) liquid sample, thereby improving metering accuracy. The seal means may be located within a distance of 40, 20, 10 or 5 bore diameters from the lancet piston tip, preferably within a distance of 2.5 or one bore diameters or less, e.g. in the before-use and/or after-use configuration.

In some embodiments, the lancet piston tip may be disposed forwardly of the seal means when the lancet piston is in the retracted position. In such cases, the lancet piston may be formed from a spike member protruding from a support, the support sealing against the internal surface of the bore. In these embodiments, the lancet piston tip itself may protrude into the liquid-containing space.

Preferably, the disposable device and/or the actuator has cooperating means for providing an intermediate delay position for the lancet piston between the puncture position and the retracted position. The intermediate delay position acts to halt the retraction of the lancet piston tip after puncturing the skin, but keeps the lancet piston tip safely out of contact with the skin. This delay allows liquid to accumulate on the skin before drawing it into the liquid containing space by further retraction of the lancet piston.

Preferably, the disposable medical device has at least one stop member for limiting the forward and/or rearward travel of the lancet piston.

At the puncture position, the seal means may be out of contact with the sealing surface, or may contact the sealing surface with reduced pressure compared with the retracted position, thereby providing low friction for movement of the lancet piston at the puncture position. During retraction from the puncture position, the contacting or increased contacting of the seal means with the sealing surface may provide the intermediate delay position set out above.

Preferably, the bore includes a region of increased cross-section dimension for location of the seal means or the sealing surface at the puncture position of the lancet piston. The seal means or sealing surface may be disposed beyond the forward end of the bore at the puncture position of the lancet piston, thus rendering the seal ineffective at the puncture position.

Preferably, the seal means is a sealing member (e.g. a piston seal) disposed rearwardly in the device from the forward tip of the lancet piston and movable with the lancet piston tip and the sealing surface is formed by the internal surface of a sealing region of the bore. Typically, a characteristic cross-sectional dimension (e.g. diameter) of the sealing member is greater than a cross-sectional dimension of the liquid-containing space but less than or equal to 10 (preferably 5, more preferably 2) times the cross-sectional dimension of the liquid-containing space.

Preferably, the lancet piston tip is sharpened to have a radius of curvature in at least one dimension of one quarter or less of the narrowest cross-sectional dimension of a non-tip region of the lancet piston. This radius of curvature is more preferably one sixth or less, one eighth or less, or most preferably one tenth or less of the narrowest cross-sectional dimension of a non-tip region of the lancet piston. '

Preferably the bore has an internal cross sectional dimension of 5 mm or less. This is a suitable internal diameter to allow a suitable quantity of blood, for example, to be retained in the liquid-containing space by surface tension and pressure differential against gravity. The bore may be narrower than 5 mm diameter, for example 4 mm or less, 3 mm or less, 2 mm or less, 1 mm or less, or 0.8 mm or less, or about 0.6 mm or less, or about 0.4mm. The sample of blood or other liquid must be of sufficient volume to bridge across and completely fill the cross-section of the bore for an axial length of at least one bore diameter, preferably more. The bore and lancet piston may be sized appropriately in diameter and length to fulfil this requirement for various aspirated volumes of sample between 1 nl and 300 µl.

The lancet piston will be sized to fit within the bore via the seal means. The cross-sectional dimension of the lancet piston (typically a non-tip region of the lancet piston) is preferably 0.1 mm or greater. More preferred ranges for this dimension are 0.2 mm or greater, 0.3 mm or greater, 0.5 mm or greater, or 0.8 mm or greater. Alternatively, the cross-sectional dimension of the lancet piston (typically a non-tip region of the lancet piston) may fall within the ranges specified above for the bore dimensions.

The volume of the liquid-containing space when the lancet piston is in the retracted position may be 0.1 microlitres or more. Preferably this volume is 0.2 microlitres or more, 0.5 microlitres or more, 1 microlitre or more, 5 microlitres or more or 10 microlitres or more. This volume is typically 300 microlitres or less. Preferably this volume is 250 microlitres or less, 200 microlitres or less, or 150 microlitres or less.

Preferably, the bore includes a chamber portion, disposed between and having a greater cross-sectional dimension than forward and rearward portions of the bore. The cross-sectional dimension of the bore may increase stepwise between the forward portion of the bore and the chamber portion.

In use, the liquid-containing space may present means for measuring or testing a characteristic of the liquid contained in the liquid-containing space, e.g. at the chamber portion.

The optional incorporation of a sensor chamber offers a means to integrate test-strip chemistry to perform electrochemical, photometric or immunological tests. Sensors may also be incorporated to enable physical measurements of blood properties to be made. Examples of the type of tests that may be integrated with the device include blood glucose concentration, HbAlc (glycated haemoglobin), cholesterol, triglycerides, blood ketone, cardiac markers, (e.g. troponin I, myoglobin, D-dimer, CK MB, BNP), osteoporosis tests, ions and electrolytes (e.g. pH, Na⁺, K⁺, Ca⁺⁺, Cl⁻) and prothrombin time (PT). It will be understood that the invention is not necessarily limited to the use of these examples and can be applied to many methods of blood analysis performed in small chambers.

Optionally, the device has a secondary conduit from the bore, communicating with the liquid-containing space. This allows connection, for example, of the bore with a reagent reservoir, e.g. in the form of a bladder. Alternatively, the secondary conduit may provide an alternate outlet for the sample contained in the liquid-containing space.

The device may have a closure member located forwardly of the lancet piston tip, for sealing at least a part of the liquid-containing space. The closure member may be adapted to be removed or punctured by the lancet piston during operation of the device. The liquid-containing space may contain a liquid for application to a subject by operation of the device. For example, the liquid may be an allergy testing liquid (allergen), anaesthetic liquid, anticoagulant liquid or an antiseptic liquid.

Preferably, the device includes a spacer member located forwardly of the forward end of the bore, the spacer member being for contact with the skin of a subject. The spacer member may be dimensioned to provide an accumulation space between a puncture in the skin of the subject and the forward end of the bore, liquid from the puncture being able to accumulate in the accumulation space before being drawn into the liquid-containing space by operation of the device. Preferably the spacer member has a transverse internal dimension (for example diameter) of 5 mm or less. The axial extent of the spacer member may be 10 mm or less. The forward end of the bore may be located within the space enclosed by the spacer member. The liquid drop accumulating on the skin surface within the accumulation space need not contact any part of the spacer member. However, in some embodiments, such contact may be suitable to encourage flow of said liquid towards the forward end of the bore. The spacer member may include one or more projections projecting inwardly towards the puncture position of the lancet piston tip. These projections may provide a further internal surface to enhance the flow of an accumulating liquid towards the forward end of the bore.

The device may include sensing means for sensing the presence or absence of liquid at the forward end of the bore. The sensing means may include at least two electrodes, at least one of which is located at the forward end of the bore to detect the presence or absence of liquid at the forward end of the bore by measurement of the resistance between the electrodes. For example, the lancet piston may provide one of the electrodes.

The device may include sensing means for sensing the presence and/or amount of liquid along the liquid-containing space. Preferably, the sensing means includes at least two electrodes, one of which may be the lancet piston tip. The other electrode may be towards the rearward end of the bore. With this arrangement, when the liquid-containing space is filled with liquid having some conductivity but a high resistance (e.g. blood), there will be a high resistance (but not an open circuit) between the electrodes when the lancet piston tip is retracted, since the liquid should still be in contact with the lancet piston tip. Knowledge of the position of the lancet piston (by observation) with respect to the bore will provide an indication of the volume of liquid in the bore since the liquid is in contact with the lancet piston tip and the bore dimensions are known. The presence or entrainment of any significant air bubble in the liquid-containing space will be indicated by a high resistance or by a jump in resistance.

Preferably, electrical connections to the aforementioned electrodes are made between the actuator and the device by means of the releasable engagement features on the device and corresponding actuator. The electronic system to take measurements from these electrodes is preferably incorporated into the re-usable actuator.

Preferably, in use of the device, the puncture operation of the lancet piston tip punctures the skin of a subject and the liquid drawn into the liquid-containing space is at least one of the blood of the subject and the interstitial fluid of the subject. Preferably, the mode of operation of the device includes the step, after puncturing the skin of the subject, of delaying retraction of the lancet piston at an intermediate delay position, where the lancet piston tip is not in contact with the skin, to allow blood or interstitial fluid to accumulate on the surface of the skin of the subject for subsequent drawing into and along the liquid-containing space.

In a preferred use of the device, there is included the step, before puncturing the skin of a subject, of expelling liquid from the liquid-containing space onto the skin of the subject at the skin location to be punctured. This provides the advantage that the liquid is applied to the skin before the puncture operation, which is of use particularly in allergy-testing applications.

In preferred uses, the device can eliminate the need to use separate devices for piercing the skin, sampling and metering the blood or liquid. Preferred embodiments of the device allow transfer of the liquid sample to a sensor or analysis chamber. Further embodiments are capable of delivering a liquid to the skin before piercing. The device may be operated under automated control.

In a preferred first use, for performing an allergy skin prick test or vaccination, the body of the actuator is engaged with and attaches to the rearward end of the lancet housing. The forward end of the bore is immersed in a test or vaccine solution and the actuator member or rod is driven forward (with respect to the body of the actuator) such that it engages with and pushes the rearward end of the lancet piston forward, causing the forward end of the lancet piston to be driven to a point near to or beyond the forward end of the bore. During or at the beginning of the forward stroke the push rod of the actuator attaches to the rearward end of the lancet piston. The push rod of the actuator is then retracted to retract the lancet piston to aspirate a volume of liquid and the forward end of the bore is withdrawn from the liquid. The forward end of the bore is then presented to and rested on the skin or held a small distance off it. The push rod of the actuator is driven forward to cause the lancet piston to expel the liquid on to the skin and then to drive the sharp tip of the lancet piston through the liquid and into the skin to puncture it. The push rod of the actuator is then retracted, causing the lancet piston to be withdrawn into the bore whereupon the apparatus is removed from the patient. The disposable medical device may then be detached from the actuator and discarded wherein the lancet piston remains housed within the bore to prevent needlestick injury. The device may, alternatively, have been filled with liquid and capped or sealed before being attached to the actuator, allowing for single use pre-packaged tests.

In a preferred second use, for sampling blood, ISF or other tissues in liquid form, the rearward end of the lancet housing is attached to the actuator as above. The forward end of the bore is presented to the skin and the lancet piston is driven forward by the push rod of the actuator, causing the sharp tip of the lancet piston to be projected beyond the forward end of the bore and to penetrate the skin to cause a wound. As before, the lancet piston attaches to the push rod of the actuator during, or at the beginning of, the forward stroke. The push rod of the actuator is then retracted to withdraw the lancet piston tip out of contact with the skin. The forward end of the bore is optionally taken off the skin or has a standoff feature as set out above. This is preferred in order to prevent a seal between the bore and the skin. After waiting for blood or ISF or other liquid to evolve from the wound, the forward end of the bore is immersed in the liquid such that it does not seal onto the skin, and the push rod of the actuator is retracted to retract the lancet piston, drawing liquid into the bore where it may held for subsequent ejection into another device or compartment, or may be transferred to sensors or a chamber for analysis within the bore itself or connecting channels. A defined volume of liquid may be aspirated by control of the length of the retraction stroke, and/or the liquid may be dispensed in one or more aliquots by control of the forward stroke of the lancet piston. The medical device may then be detached or ejected from the actuator whilst the actuator member is held fixed or locked at its fully retracted after-use position and discarded wherein the lancet piston remains entirely housed within the bore of the disposable medical device to prevent needlestick injury. The device may also deliver liquids by positive displacement to the skin before lancing.

In both uses described above the actuator may be driven manually, by spring, hydraulic, pneumatic, electrical force, or with the aid of a motorised, automated system.

Disclosed herein is a method of operating a medical apparatus, the apparatus consisting of a re-usable actuator with a body incorporating a feature at its forward end to releasably attach to the rearward end of the lancet housing of a disposable medical device, a push rod incorporating a feature at its forward end to releasably attach to the rearward end of the lancet piston of a disposable medical device, said push rod being capable of forward or rearward movement in relation to said body, a disposable medical device having a lancet housing with a bore and a lancet piston slidably and sealably fitting in the bore, the lancet housing incorporating a feature at its rearward end for releasably attaching to said body of the actuator, the lancet piston incorporating a feature at its rearward end for releasably attaching to said push rod of the actuator, the lancet piston being moveable relative to the lancet housing by means of movement of the push rod of the actuator between a puncture position, in which a sharpened tip of the lancet piston is exposed from a forward end of the bore for a puncture operation, and a retracted position, in which the lancet piston is rearwardly displaced along the bore to define a liquid-containing space in the bore forwardly of the lancet piston tip, the liquid-containing space having a oross-seotion dimension of 5mm diameter or less to allow liquid such as blood, ISF or other aqueous solution or suspension to be retained therein by a combination of surface tension and a partial negative pressure, the device having seal means operable substantially to prevent flow of liquid from the liquid-containing space and to prevent air flow into the liquid-containing space past the seal means on movement of the lancet piston, the method including one or more of the following steps:
(i) providing the disposable device with the lancet piston retracted towards the rearward end of the bore in the lancet housing,
(ii) presenting the actuator to the disposable medical device where the push rod of the actuator is already retracted towards the rearward end of the actuator,
(iii) attaching the body of the actuator to the lancet housing of the medical device,
(iv) attaching the push rod of the actuator to the rear of the lancet piston at the beginning of, or during, the forward stroke
(v) driving the push rod of the actuator in the direction of the actuator's forward end to push on the rearward end of the lancet piston, thus displacing the lancet piston towards the puncture position, expelling liquid that is optionally contained within the bore of the medical device,
(vi) retracting the push rod of the actuator to retract the lancet piston tip back within the forward end of the bore,
(vii) optionally pausing retraction,
(viii) further retracting the push rod of the actuator towards the rearward end to retract the lancet piston tip further back within the bore thus aspirating liquid that is in contact with the forward end of the bore,
(ix) optionally, displacing the push rod of the actuator towards the forward end to expel liquid within the bore, before retracting again as in (vi) to (viii)
(x) disengaging the push rod of the actuator from the rear of the lancet piston to leave the lancet piston retracted within the bore
(xi) disengaging the body of the actuator from the rear of the bore to release or eject the medical device.

Steps (x) and (xi) may or may not occur simultaneously. These steps may be used in combination to allow for the desired functions of:
1. Aspirating a liquid (e.g. solution such as allergen solution) from a reservoir, then dispensing said liquid onto the skin before lancing through the liquid.
2. Providing a lancet piston/lancet housing/bore device pre-filled with solution, then dispensing said solution onto the skin before lancing through the liquid, retracting the lancet piston to a safe position and releasing or ejecting the used device from the actuator.
3. Lancing the skin, retracting the tip out of contact with the skin for safety, pausing to allow blood or ISF or liquid sample to collect as a droplet, contacting the forward end of the bore with the droplet such that the sample liquid seals and bridges completely across the forward end of the bore, aspirating a sample of liquid and dispensing all the liquid or an aliquot of it before retracting the lancet piston to a safe position and releasing or ejecting the used device from the actuator with the lancet piston remaining in a safe position, completely inside the lancet housing.
4. Providing a lancet piston/lancet housing/bore device pre-filled with solution, then dispensing said solution onto the skin before lancing through the liquid, lancing the skin, retracting the lancet piston tip just within the forward end of the bore out of contact with the skin for safety, pausing to allow blood or ISF to collect as a droplet, contacting the forward end of the bore with the droplet if it is not already in contact, aspirating a sample of liquid and dispensing all the liquid or an aliquot of it before retracting the lancet piston to a safe position and releasing the used device with the lancet piston remaining in a safe position.
or any combination of these steps.

Preferably, the bore, or, more generally, the inner surface of the lancet housing, incorporates one or more features to hold the lancet piston in one or more retracted positions wherein the lancet piston tip does not project beyond the forward end of the bore when the disposable device is detached from the actuator during storage and after use.

Preferably, the axial length of the lancet piston is shorter than the axial length of the lancet housing such that the rearward end of the lancet piston does not protrude from the rearward end of the lancet housing at any time during normal use and after it has been ejected from the actuator.

Preferably, the rearward end of the lancet housing and the forward end of the body of the actuator are attached by means of a releaseable snap fit or interference fit.

Preferably, the forward end of the push rod of the actuator has a set of normally sprung-open clutch jaws, the rearward end of the lancet piston incorporating a gripping feature that the clutch jaws can envelop. The lancet housing may have an internal guide feature against which eternal faces of the clutch jaws can bear to co-operatively close the clutch jaws around the gripping feature on the rearward end of the lancet piston during forward motion, said jaws being arranged to open to release the gripping feature on retraction of the actuator push rod to an axial position where the clutch jaws are opened sufficiently by contact with the guide feature to release and detach from the gripping feature at the rearward end of the lancet piston. The guide feature may be an inwardly-tapering inner surface of the lancet housing.

The actuator incorporates a mechanism to actively close the set of normally sprung-open clutch jaws. The normally sprung-open jaws may, for example, be of the kind commonly used in propelling pencils, being a rearwardly-tapered hollow cylinder out into two or more radial segments for part of its length from the open end to form the sprung members of a clutch. In the open position (before use), the radial jaws are sprung outward. On retraction of the sprung jaws into a cylindrical housing under spring pressure the taper acts as a wedge to close the jaws. The inside diameter of the clutch is slightly smaller than the outside diameter of the feature it is intended to grip.

In one embodiment of the present invention the sprung jaws are mounted on an inner shaft. An outer hollow cylindrical shaft surrounds the inner shaft and has an internal diameter larger than the smallest diameter at the rear of the tapered clutch jaws and smaller than the fully closed diameter of the sprung jaws. Moving the outer cylindrical shaft forward with respect to the inner shaft forces the jaws shut as the hollow cylindrical shaft bears against the rear taper of the jaws to grip the lancet piston. Both shafts are arranged to advance together once the lancet piston is gripped. On retraction of the lancet piston to the after-use position, the outer cylindrical shaft is retracted relative to the inner shaft to open the jaws and disengage from the lancet piston. This mechanism differs from that of a propelling pencil because in a propelling pencil the outer cylinder remains fixed with respect to the body of the device (pencil housing) and the jaws are pushed forward to open. Such a mechanism is suitable for feeding a pencil lead forward and then gripping it again. In the above described embodiment of the present invention the outer cylinder is movable with respect to the actuator and this allows the clutch to be closed around the rear of the lancet while the jaws and lancet piston remain in a fixed axial position relative to the actuator and lancet housing, and thereafter for the whole clutch to move with the lancet piston.

The lancet piston housing may have a hollow cylindrical section forward of the before-use position of the rearward part of the lancet piston, this hollow cylindrical section of the housing having an internal diameter marginally larger than the outer diameter of the clutch when gripping the lancet piston. This cylindrical section of the lancet housing is at least the same length as the desired stroke of the lancet piston. This cylindrical section acts as a secondary interlock. When the clutch jaws are within this cylindrical section they are prevented from opening and thus releasing the lancet piston accidentally.

In preferred embodiments and uses, the apparatus provides for a disposable device of minimal size and cost together with a re-usable actuator wherein the lancet tip is shrouded before and after use and can be loaded onto and ejected from the actuator without risk of injury to the user.

In a further preferred use, the apparatus can eliminate the need to use separate devices for piercing the skin, sampling and metering the blood or liquid. Preferred embodiments of the apparatus allow transfer of the liquid sample to a sensor or analysis chamber. Further embodiments are capable of delivering a liquid to the skin before piercing. The apparatus may be operated under automated or sequenced control.

### BRIEF DESCRIPTION OF THE DRAWINGS

Examples of the invention will now be described by way of example with reference to the accompanying drawings, in which:
Figure 1 is a sectional view of a device suited to the delivery of liquids to the skin before piercing the skin, the device being for comparison with embodiments of the invention;
Figure 2 is a series of sectional views of the device in Figure 1 being applied to a sequence of operations
Figure 3 is a sectional view of an apparatus to drive the device of Figure 1 in use;
Figure 4 illustrates three stops in the use of the device of Figure 1 with the apparatus of Figure 3 for allergy testing;
Figure 5 illustrates a sectional view of a medical device outside the scope of the invention suitable for both allergy testing and blood sampling;
Figure 6 illustrates the device of Figure 5 engaged with an actuator in use at one step in a sequence;
Figure 7 illustrates the device and actuator shown in Figure 6 in use at one step in a sequence;
Figure 8 illustrates the device and actuator shown in Figure 6 in use at one step in a sequence;
Figure 9 illustrates the device and actuator shown in Figure 6 in use at one step in a sequence;
Figure 10 illustrates the device and actuator shown in Figure 6 in use at one step in a sequence;
Figure 11 illustrates a device and actuator wherein an actuator is shown in elevation view and the forward part of the actuator with attached disposable device is shown in sectional view (the latter as in Figures 6-10);
Figure 12 is an illustration of a single use medical device and an actuator outside the scope of the invention integrated with a Point of Care meter system;
Figure 13 illustrates a single use medical device and an actuator outside the scope of the invention with a sprung engagement means on the lancet piston;
Figure 14 illustrates an embodiment of the invention with features on the actuator to actively close the sprung engagement means onto the lancet piston;
Figure 15 illustrates an embodiment of the invention shown in Figure 14 at a second stage of operation;
Figure 16 illustrates an example actuator mechanism to actively close the clutch shown in Figures 14 and 15.

### DETAILED DESCRIPTION OF PREFERRED EMBODIMENTS

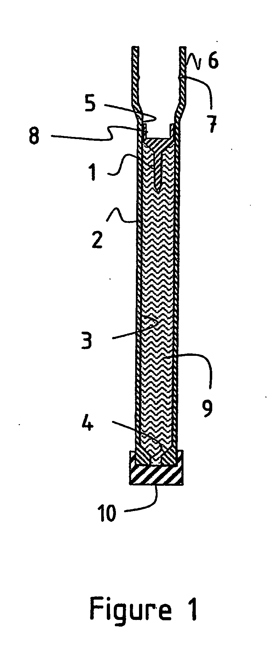

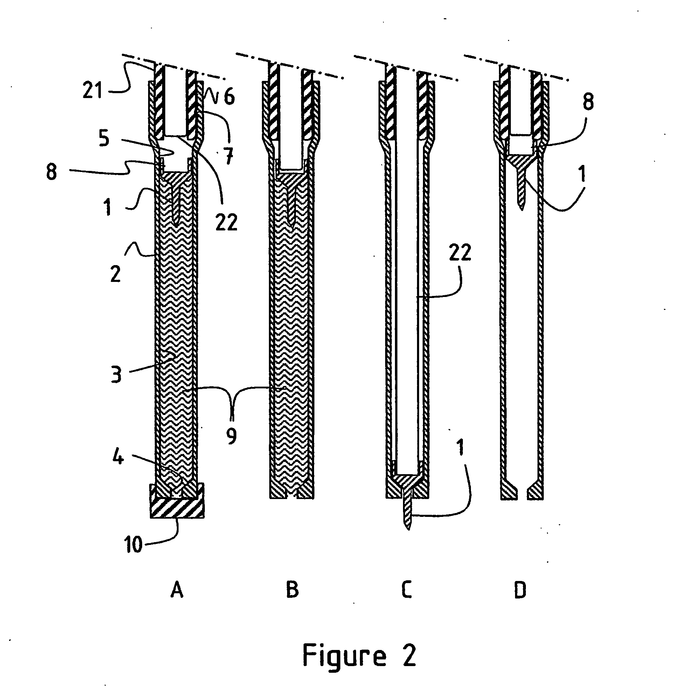

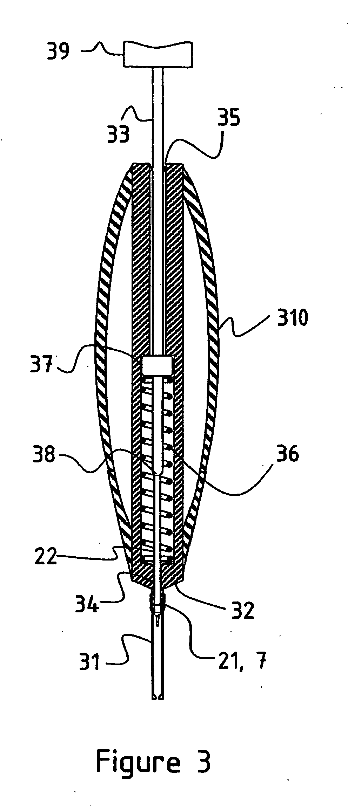

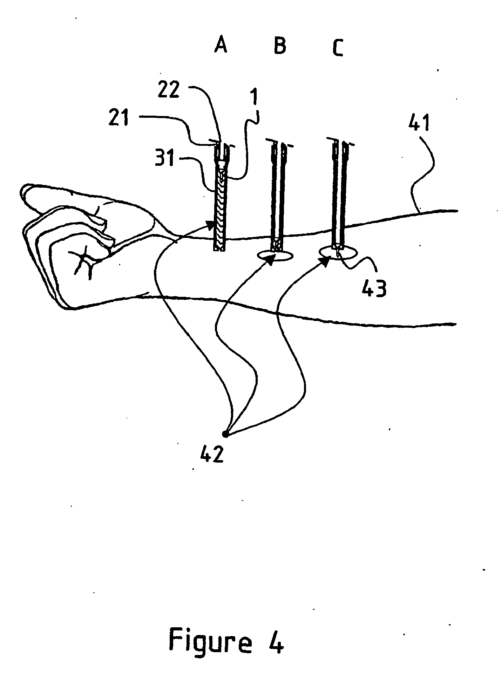

Figures 1-4 illustrate a device and its use that is of interest to compare with embodiments of the invention. This device is also described in an earlier patent application (PCT/GB2005/003534, publication number W02006/030201) by the present inventors. Figure 1 illustrates a disposable device that is suited to the delivery of liquid to the skin before puncture of the skin through the dispensed liquid. Figure 2 shows this disposable device being driven by the forward end of an actuator. Figure 3 illustrates the complete actuator, and Figure 4 illustrates how liquid is delivered to the skin before piercing for an allergy test.

Figure 1 illustrates a device consisting of a lancet piston 1 housed in a bore 3 within a lancet housing 2. The whole of the lancet housing 2 and lancet piston 1 represents a disposable (single use) device for aspirating and dispensing liquid and piercing the skin. The lancet piston 1 can be constructed of a single material as illustrated, or may be an assembly or single part consisting of a lancet portion, piston seal and plunger (not shown). The lancet piston 1 has a socket 8 at its rearmost end and is contained within a bore 3 in the lancet housing 2. The forward end 4 of the bore 3 is reduced in diameter to provide an end-stop to limit forward travel of the lancet piston 1 and to assist in retaining liquid within the bore. The rearward end of the lancet housing has an attachment feature 6 incorporating a snap-fit feature 7 to connect with the forward end of an actuator (not shown) for driving the lancet piston. The feature for attaching the lancet housing to the actuator could equally be a press-fit, twist lock or other fixing. The bore 3 may be filled with a liquid 9 and sealed with a removable cap 10. The assembly of Figure 1 illustrates an example of a disposable device pre-filled with liquid.

Figure 2 illustrates the disposable device of Figure 1 being used to deliver liquid to the skin before piercing through said liquid, being driven by an actuator (only the forward part of the actuator is illustrated). Configuration A shows the device before use, with the bore forward of the lancet piston filled with a liquid 9 for delivery to the skin. A removable cap 10, together with the lancet piston seal at the rear of the device, retains the liquid, preferably in a sterile condition, before use. The attachment feature 6 and snap fit 7 of the lancet housing 2 are shown releasably attached to the forward part of the body of an actuator 21. This actuator incorporates a push rod 22 that can slide in relation to the body of the actuator, only the forward end of this push rod being illustrated here. In configuration B the cap 10 has been removed. The push rod 22 is extended and engages with the socket 8 in the rear of the lancet piston 1. Further extension of the push rod drives the lancet piston 1 down the bore 3, expelling the liquid 9. Configuration C shows the lancet piston at the forward end 4 of the bore 3 with the tip of the lancet piston 1 projecting to puncture the skin. At this point the push rod 22 push-fits into and securely engages with the socket 8 of the lancet piston 1 if it has not already done so. Retracting the push rod 22 carries the lancet piston 1 back up the bore 3 until it comes to rest against the forward end of the body of the actuator 21. Configuration D illustrates further withdrawal of the push-rod 22 to release the lancet piston 1 from the push-rod in a safe position where the lancet point is shielded within the bore, protecting users from needle-stick injuries. Optionally, a rearward end-stop feature (not shown), similar to snap-fit feature 7, may be formed on the bore wall at the retracted position to ensure that the lancet piston 1 cannot be removed from the rear of the bore 3. The lancet housing 2 containing the lancet piston 1 may now be detached from the forward end of the actuator 21 and discarded.

Figure 3 illustrates a cross-section through a hand-held actuator for driving the device of Figures 1 and 2 through the steps of aspirating, dispensing and piercing. For ease of explanation, the rearward end of the actuator is toward the top of Figure 3. The actuator consists of an inner casing 32 enclosing a compression spring 36. A push rod 33 passes down through the inner casing 32 and is guided by a rearward guide 35 and a forward guide 34, both of these guides providing a low-friction sliding fit. The rearward end of the pushrod 33 is fitted with a thumb button 39. A spring seat 37 is attached to the push rod 33 and slides freely within the inner casing 32. A disposable device 31 consisting of a lancet piston and bore assembly of the type shown in figure 1 is shown fitted to the forward end 21 of the actuator body 32. The device 31 in Figure 3 is shown at about actual size, whereas the same device in Figures 1,2 and 4 are shown greatly magnified for clarity. When the thumb button 39 is depressed the forward end 22 of the push rod 33 is projected beyond the forward end 21 of the inner casing 32 and the sliding spring seat 37 compresses the spring 36 against the inner casing 32. Forward motion of the push rod 33 is limited by the step in diameter 38 in the push rod 33, being larger than the diameter of the forward push-rod guide 34. Releasing pressure on the thumb button 39 allows the push rod 33 to retract under spring pressure, the sliding spring seat 37 acting against the inner casing 32 to provide a back-stop to limit rearward travel of the push-rod 33. An outer casing 310 is provided to enable the actuator to be held easily in the hand.

It can readily be appreciated that the actuator of Figure 3 could be equipped with adjustable forward and rearward dead-stops to adjust the depth of penetration of the lancet piston and to adjust the aspirate/dispense volume. For example, adjusting the limit of the forward travel of the forward end 22 of the push rod 33 would adjust the depth of penetration of the lancet piston point. Adjusting the limit of the rearward travel of the forward end 22 of the push rod 33 would adjust the swept volume of the device. It can further be appreciated that the example illustrated in Figures 1-3 can be adapted to allergy tests. The bore may be filled with allergen or a suspension of allergen particles in a carrier liquid, control liquid or suspension by aspiration either during manufacture (and sealed in the bore), at the point of use from a bulk container or bottle containing allergen, or at the point of use from a transfer station or well which has been pre-filled with a small amount of test solution or suspension from a bottle of allergen.

An example of how the device of Figure 1 may be used for allergen testing is illustrated in Figure 4. The device 31 is the same as that of Figures 1-3. For ease of illustration it is not to scale with the patient's arm. It is fitted onto and operated by the actuator of Figure 3. For ease of illustration, only the forward part 21 of the inner casing 32 from Figure 3 is shown. The operation is as follows:
The bore of the device 31 is filled with allergen 42 by the method described previously in PCT/GB2005/003534 by the present inventors. Alternatively, if the device 31 has been pre-filled with allergen solution as in Figure 2 configuration A, then remove the end cap shown in Figure 2 as feature 10;
Figure 4 sequence A: Lightly place the forward end of the device 31 onto the skin 41 so that the disposable device 31 is held near perpendicular to the skin surface 41 and does not form a pressure-tight seal onto the skin (i.e. any seal to the skin is easily overcome by fluid pressure generated by forward movement of the lancet piston);
Figure 4 sequence B: Dispense the allergen sample 42 onto the skin 41 by depressing the thumb button on the actuator shown in Figure 3 as feature 39;
Figure 4 sequence C: Continue with further forward movement of the thumb button to extend the lancet piston 1 beyond the end of the bore device 31 such that the tip 43 of the lancet piston 1 passes through the dispensed allergen sample 42 and punctures the skin 41;
The sequence is completed by releasing thumb pressure on the actuator (feature 39 in Figure 3), withdrawing the lancet piston 1 so that the lancet piston tip 43 is safely rehoused in the bore, and removing the disposable device 31 from the skin surface 41. This may aspirate a volume of the allergen sample 42 and may or may not leave a portion of the allergen sample 42 on the skin surface at the puncture site (not shown).

Figures 1-3 are intended to illustrate one example of an actuator-and-device combination. The feature for connecting the lancet housing to the actuator can be of any suitable form, for example push fittings, snap fittings, collets, mechanical clutches, twist-lock fittings or any of the many connection fittings found in the arts of medical devices and laboratory pipettors.

It can be appreciated that the example of the combination of the disposable device of Figure 2 and actuator illustrated in Figure 3 is also capable of aspirating a liquid. Referring to Figure 2, the push rod 22 may be fully extended as at configuration C to engage the lancet piston 1. The forward end 4 of the bore 3 may then be immersed in a liquid and the push rod 22 retracted to a position approximating configuration B. This action will fill the bore with liquid 9. The liquid may be delivered to the skin by reversing the push rod 22 to expel the liquid. The combination of device and actuator may also be used to pierce the skin and aspirate a blood sample. Stating from configuration D the lancet piston 1 is driven down the bore to configuration C to pierce the skin. At this point the lancet piston 1 can be retracted slightly so that the piercing point of the lancet piston 1 is recessed just inside the forward end 4 of the bore 3 (position not shown), Once a droplet of blood has formed on the akin, the forward and 4 of the bore 3 may be immersed in the blood droplet and a sample of blood may be aspirated by further withdrawing the lancet piston 1.

This example (described in Figures 1-4) has several limitations in use. The push rod will only grip the socket on the rear of the lancet piston securely when the lancet piston reaches the end of its travel because the lancet piston is only restrained within the bore by friction. As the push rod is extended into the socket at the rear of the lancet piston, the lancet piston will move forward. If the push rod is retracted before it has gripped the sooket firmly, the lancet piston may not be retracted with it, and the lancet piston tip may be left in an exposed position. Furthermore, the user is likely to experience an unpleasant (and potentially painful) jolt when the push rod is pushed fully home because the lancet piston will already have pierced the skin at this point. It may also be difficult to arrange for the force required for the push rod to push fit into the lancet piston socket to be less than that needed to push the bore off the actuator. It is also possible that the lancet piston may stick in the patient's skin or that the frictional force between the lancet piston seal and the bore exceeds the grip force at the socket, resulting in detachment of the push rod from the lancet piston when retracting the lancet piston from the exposed position.

What would be more appropriate is an engagement mechanism that can securely engage the lancet piston at a first retracted position without moving the lancet piston an appreciable distance down the bore, and in a way that ensures the lancet piston cannot be left behind if the push rod is retracted before the end of its travel. We now describe a device that offers such an improvement over the example above.

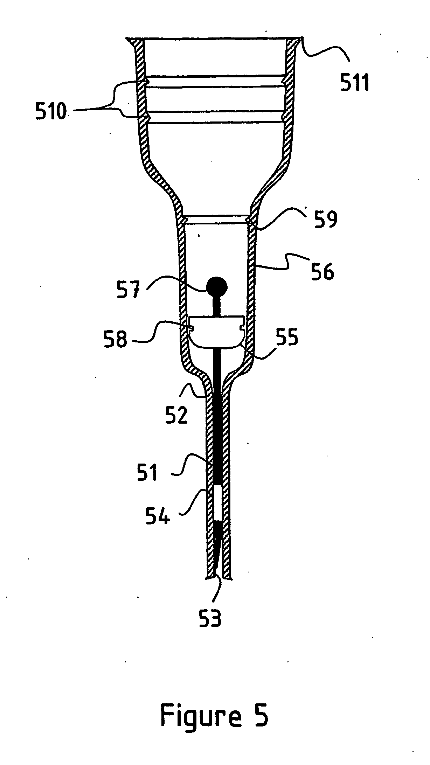

A first device is shown in Figures 5-10. This is outside the scope of the invention. With reference to Figure 5, a disposable device has a lancet piston 51 with a piston seal 54. The piston seal is housed in a bore 53 formed in the lancet housing 52. The piston seal may provide sufficient friction to prevent unintended axial movement of the lancet piston relative to the lancet housing during storage and use. The close fit between the lancet piston 51 and the bore 53 also serves to constrain the radial position of the rear feature 57 of the lancet piston

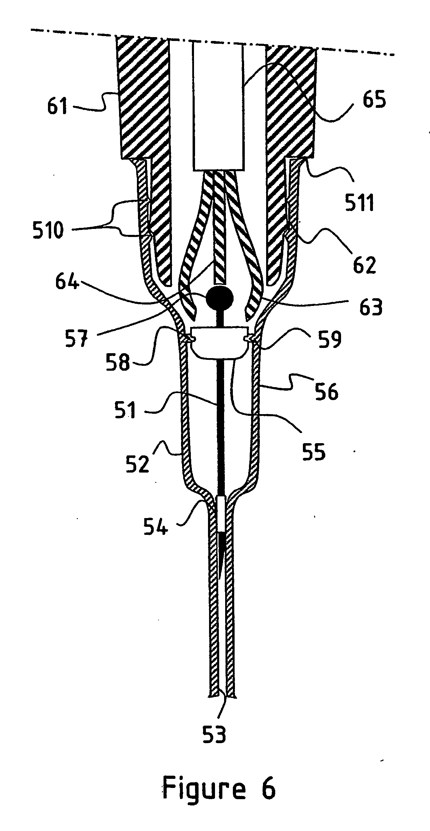

The lancet piston has a guide feature 55 towards the rearward end of the lancet piston. This guide feature may incorporate a detent feature 58 (symmetrical, one side only labelled for clarity), this feature being shown as a recess. At the extreme rearward end of the lancet piston is a feature 57 for releasably attaching to a clutch on an actuator (not shown). The lancet housing 52 incorporates a region of increased diameter 56 (relative to the bore 53) that is larger in diameter than the lancet piston guide 55 to allow the guide feature to slide freely within this region. The lancet housing also incorporates a bump-off feature 59 that is able to retain the lancet piston in a rest position by engaging with the corresponding detent feature 58 on the lancet piston guide (see Figure 6). Features 58 and 59 are shown as sharp-edged features, but clearly these may be replaced by an area of increased frictional contact between the lancet piston guide 55 and the bore section 56 at or near to the retracted park position (not shown) Towards the rearward end of the lancet housing there are two annular bump-off or snap-fit features 510 for releasably engaging with the forward end of an actuator housing (not shown). The rearward end of the lancet housing incorporates an annular lip 511.

Figure 6 illustrates the assembly of Figure 5 attached to the forward end of an actuator (only the forward part is shown). The forward part of the actuator body 61 is engaged with the bump-off features 510 of the lancet housing 52 by means of a corresponding detent feature 62 on the actuator body. The annular lip 511 of the disposable device rests against the forward end of the actuator body. The actuator incorporates a push rod 65 terminated at its forward end with a spring clutch 63 (symmetrical leaves shown) and a rigid pin 64. The lancet piston 51 is shown in a parked position where the detent feature 58 in the lancet piston guide 55 is engaged with the corresponding bump-off feature 59 on the lancet housing. In this position the tip of the lancet piston is safely housed within the bore 53. The rearward end of the lancet piston is also shielded by the rearward end of the lancet housing from being accidentally extended when the actuator is not engaged with the actuator. It can be appreciated that the disposable device could be offered to the actuator with the section of bore 53 forward of the lancet piston tip already filled with liquid (not shown).

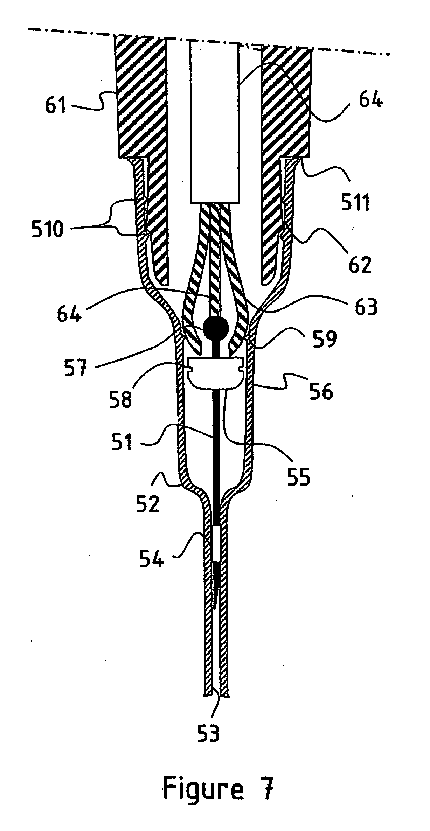

Figure 7 shows the push rod in a partly extended position. The pin 64 pushes at the rear 57 of the lancet piston 51 and displaces the lancet piston downward to push the lancet piston guide 55 off the detent and bump-off features 58 and 59. The jaws of the clutch 63 bear on the bump-off feature 59 and the guide region 56 of the lancet housing and are thus guided to compress radially inwards to encompass the feature 57 at rearward end of the lancet piston.

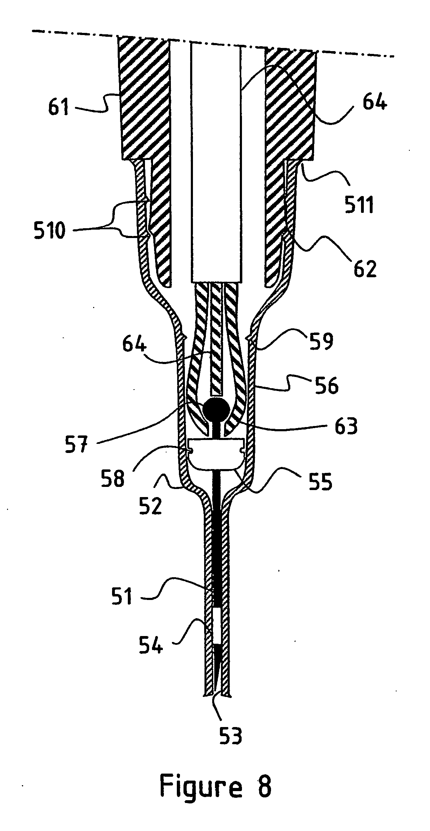

Figure 8 shows a further step in the sequence where the jaws of the clutch 63 have enveloped the feature 57 at the rear of the lancet piston. In this position the clutch cannot release the lancet piston in the forward or rearward direction, nor can it do so in any position other than at or near to the retracted park position. This is au important feature to prevent needlestick injury.

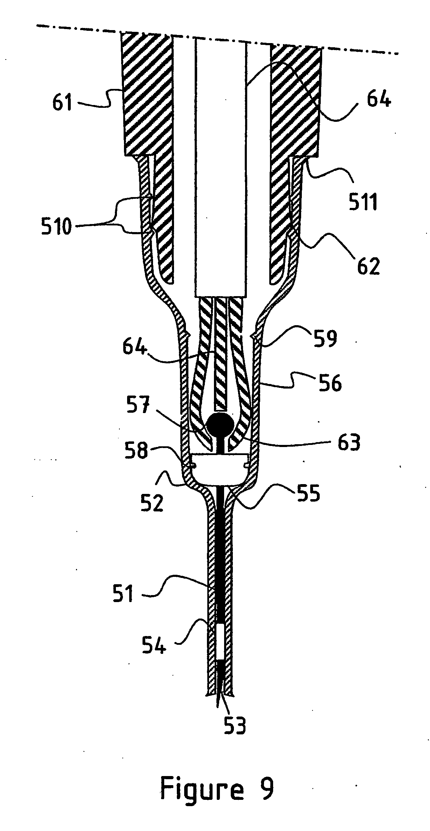

Figure 9 illustrates the lancet piston 51 at the furthest extent of forward travel, limited by the lancet piston guide 55 contacting the bottom of the guide region 56 of lancet housing 52. The tip of the lancet piston extends a short distance beyond the bore. Alternatively, the jaws of the clutch 63 may be sized and configured (not shown) to contact the bottom of the guide region 56 to limit the forward travel of the lancet piston, if the lancet piston guide 55 is not present. The lancet piston guide may be omitted where the guidance, friction and close fit between the lancet piston 51, piston seal 54 and the bore 53 Is sufficient alone to maintain the desired axial and radial position of the lancet piston during storage or before or during use.

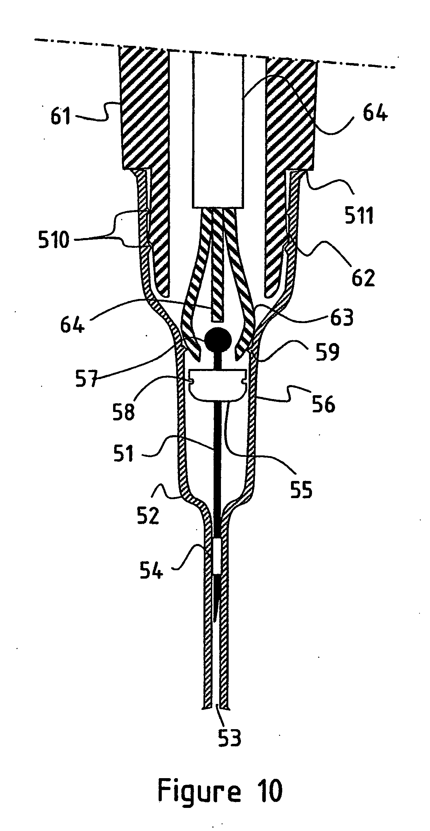

Figure 10 illustrates the retraction of the push rod to a point where the clutch 63 releases the lancet piston 51. The clutch releases the lancet piston before it has travelled all the way back up the bore. The bump-off feature 59 in combination with the lancet piston guide 55 provides an extra safety measure to prevent the lancet piston being drawn out of the lancet housing if it does not disengage cleanly from the clutch. The lancet housing 52 may be disengaged from the actuator with the lancet piston tip safely shrouded within the bore. The device can incorporate a feature to look the lancet piston at this point in the bore (not shown), however in practice the friction of the piston seal 54 against the bore 53 is sufficient to do this

The device illustrated in the sequence Figures 6-10 can be used for allergy testing. To do this the disposable device may be presented to the actuator with the lancet piston in the position shown in Figure 6 and the bore forward of the lancet piston tip already filled with allergen liquid. Figures 7-10 would then represent the sequence:
(i) dispensing the allergen (Figures 7 and 8)
(ii) piercing the skin (Figure 9)
(iii) retracting the lancet piston to the after-use position to allow disengagement of the clutch (Figure 10)
(iv) ejecting the disposable device (not shown)

Where the disposable device is not pre-filled, an alternative sequence is:
(i) offer an empty device to the actuator with the lancet piston in the before-use position (Figure 6)
(ii) immerse the forward end of the bore in allergen solution (not shown)
(iii) drive the lancet piston to the position shown in either Figure 8 or 9
(iv) retract the lancet piston to aspirate liquid (Figure 10)
(v) remove the bore from the liquid (not shown)
(vi) place the forward end of the bore on or just above the skin (not shown)
(vii) drive the lancet piston to dispense the liquid and extend the lancet piston tip to pierce the skin (Figure 9).
(viii) retract the lancet piston to the after-use position (Figure 10), where the clutch has disengaged from the lancet piston
(ix) eject the disposable device (not shown)

This device can also be used for blood sampling by varying the sequence:
(i) offer an empty device to the actuator, with the lancet piston in the before-use position (Figure 6)
(ii) place the forward end of the bore on or just above the akin (not shown)
(iii) drive the lancet piston to its forward limit to pierce the skin (Figure 9)
(iv) retract lancet piston tip out of contact with the skin, profitable with the lancet tip housed safely just inside the bore
(v) wait for blood or ISP to evolve from the wound
(vi) immerse the forward and of the bore in the drop of blood or ISF on the skin, ensuring that the blood or ISF sample bridges completely across the forward end of the bore
(vii) retract the lancet piston further to aspirate blood or ISF (Figure 10)
(viii) remove the bore from the blood or ISF (not shown)
(ix) drive the lancet piston to dispense the blood or ISF or part of it to a desired location (Figure 8 or 9)
(x) retract the lancet piston to the after-use position, where the clutch has disengaged from the lancet piston (Figure 10)
(xi) eject the disposable device (not shown)

Figures 5-10 show a feature on the end of the lancet piston that is enclosed by the jaws of the clutch. This is desirable for interlocking the clutch to the lancet piston. It is also possible to arrange a clutch to grip a plain cylindrical section at the rear of the lancet piston. According to the preferred emdodiments of the present invention such a clutch is closed by a mechanism contained within the actuator.

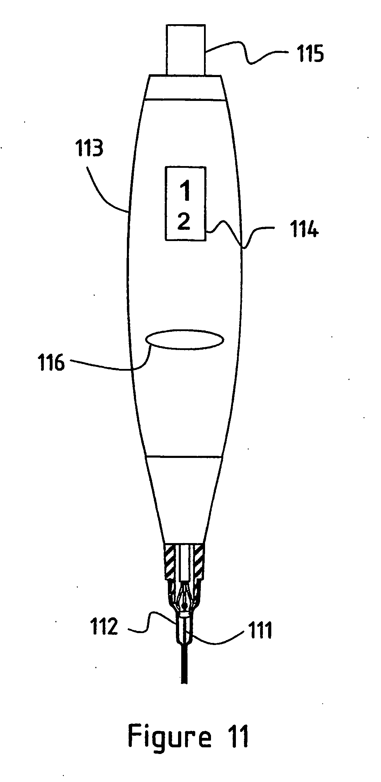

Figure 11 (outside the scope of the invention) illustrates a disposable device of the type shown in Figures 5-10 attached to a hand-held actuator. The lancet housing of the disposable device 112 (illustrated in section view) is releasably attached to the actuator 113 by a snap fit. The lancet piston 111 is shown at the befora-use position. The actuator incorporates a thumbwheel volume-setting device 114 to control the travel of the push rod, for example by means of an intermediate forward or rearward stop (not shown), a thumb-button 115 and a trigger button 116. It is envisaged that in some applications of the invention it may be desirable to integrate a spring-loaded mechanism into the actuator or the disposable for accelerating the lancet piston through at least the final stage of its forward travel to pierce the akin, such as those used in lancing devices, and this may be released by a trigger mechanism attached to the thumb button 115 or the trigger button 116.

It is also envisaged that the actuator of the present invention may be equipped with a tip-ejector of the type commonly found on conventional laboratory pipettors so that the disposable device may be pushed off or ejected from the actuator when desired. This would allow one-handed operation of the entire sequence of pick-up, engagement, use, disengagement and safe ejection of the disposable tip as described in the above description of operation of the device for various applications.

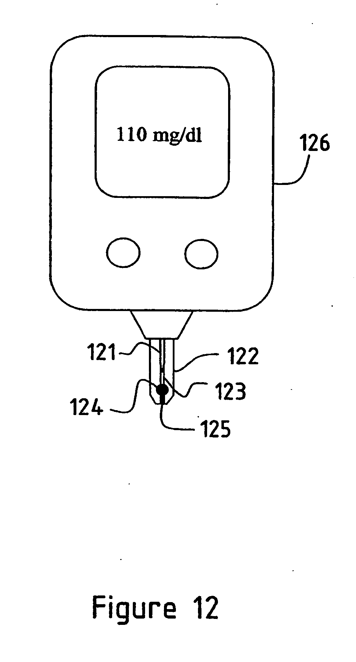

The disposable device containing the lancet piston and bore may be combined with a diagnostic test, such as a lateral flow device or blood glucose or similar test. Figure 12 (outside the scope of the invention) illustrates one embodiment where the lancet piston 121, bore 123 and a test chamber 124 are integrated into a single disposable device 122. A clutch or attachment mechanism according to the invention, such as those shown in Figure 2 and preferably those shown in Figures 6-10, can be combined with a POC meter 126 to allow the blood sampling device to be releasably attached to the POC meter and for a mechanism contained within the POC meter to drive the lancet piston to pierce the skin and aspirate a sample of blood or ISF 125 into the chamber. The disposable lancet piston/test strip combination 122 may be easily picked up and released with the lancet piston safely housed inside the bore as previously described.

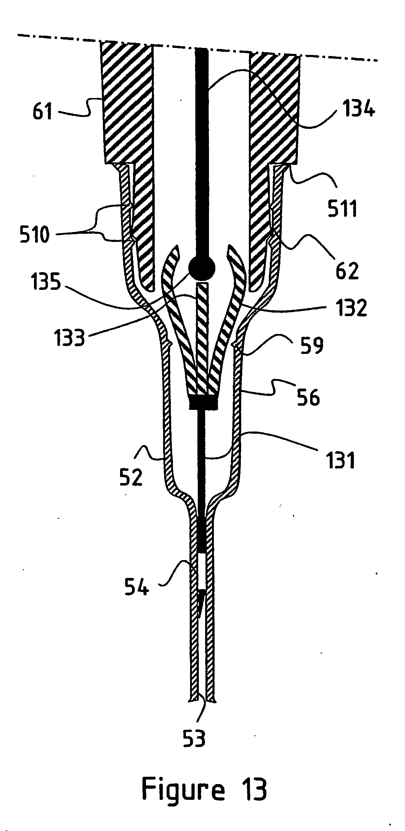

Figure 13 (outside the scope of the invention) illustrates a second device where an engagement means, being a sprung clutch, is arranged on the lancet piston itself. The lancet housing 52 and features 53, 56, 59, 61, 62, 510 and 511, together with the piston seal 54 are identical to those of Figures 5 to 10. The lancet piston 131 incorporates a spring clutch 132 and pin 133. The push rod 134 of the actuator is terminated with an attachment feature 135. Extending the push rod pushes the lancet piston towards the forward end of the bore 53 and causes the clutch jaws to react against the guide section 56 of the lancet housing 52 whereupon they envelope the feature 135 on the end of the push rod 134. This arrangement has the potential advantage that the sprung clutch on the lancet piston (which could be a plastic feature) could be arranged to retain the lancet piston in a before-use position.

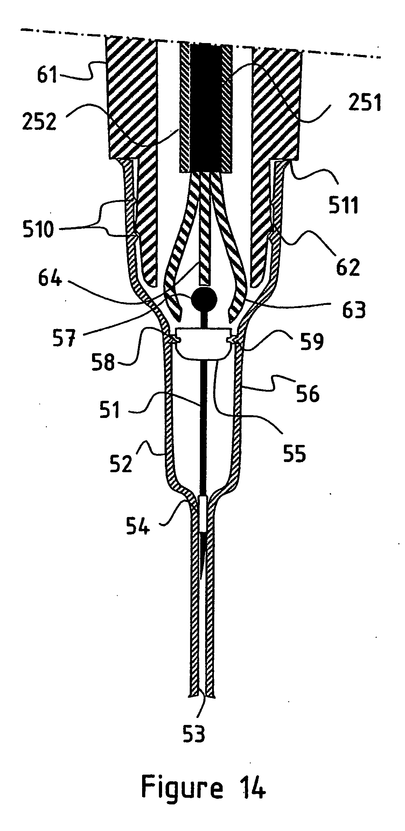

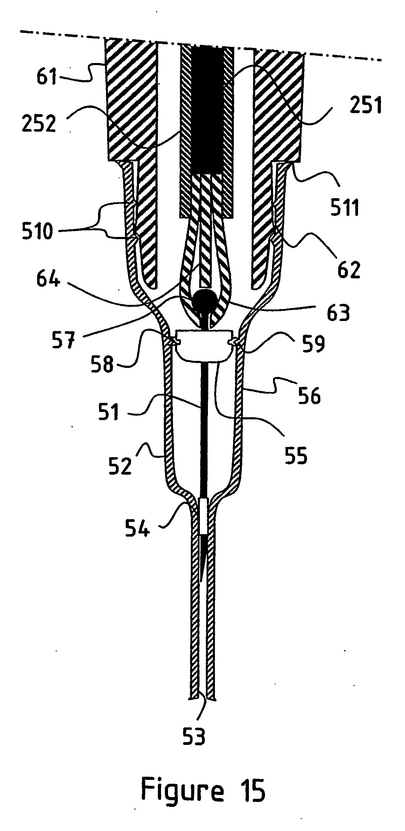

Figures 14 and 15 give an example according to the present invention of an actively-closed configuration. The push rod is arranged as two concentric parts: an inner solid shaft 251 and an outer hollow cylindrical shaft 252. The clutch jaws 63 are attached to the inner push rod 251. Figure 14 shows the clutch arrangement in the open position. Figure 15 shows the clutch closing onto the lancet piston. Advancing the outer push rod 252 forward towards the forward end of the apparatus while holding the clutch jaws 63 at a fixed axial position by means of the inner push rod 251 actively closes the jaws onto the lancet piston, providing strong mechanical grip. This would also be suitable for providing a frictional grip onto a plain cylindrical section at the rear of the lancet piston.

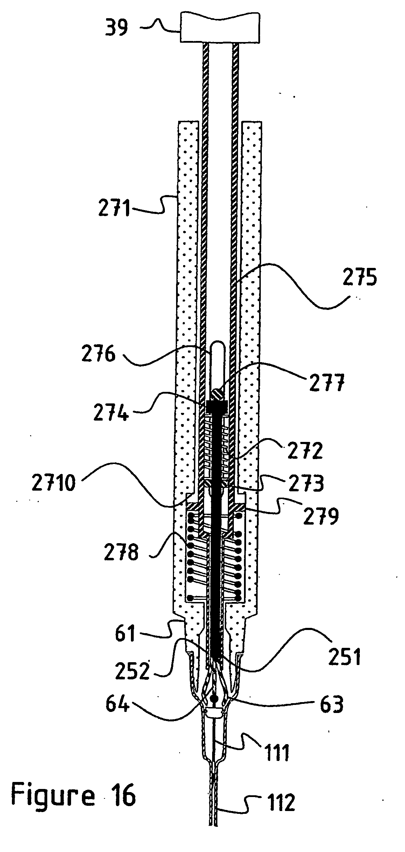

Figure 16 illustrates an example of a manually powered actuator to drive the inner solid shaft and outer cylindrical shaft of the clutch in the examples in Figures 14 and 15. A disposable device consisting of a lancet assembly 111 and lancet housing 112 is identical to that of Figures 5-11, 14 and 15 and is snap-fitted to the forward part 61 of an actuator housing 271. The olutch consisting of the centre pin 64 and sprung members 63 is attached to the inner shaft (inner push rcd) 251 as before. The inner push rod 251 is biased towards the rearward part of the actuator by moans of a strong compression spring 272 acting between a spring seat 273 (which is a fixed inward extension of the actuator housing that partially or completely encircles and guides the inner push rod 251) and a spring seat 274 which is fixed to or is part of the rearward end of the inner push rod 251 and thus fixedly moveable with it. The outer hollow cylindrical shaft 252 forms the forward part of an outer push rod 275 terminated with a thumb button 39. The rearward part of the outer push rod 275 need not be cylindrical, but must be slotted as shown by slot feature 276 to clear the support for the spring seat 273 and the dead stop 277, both fixed to the actuator housing 271. The dead stop 277 limits the rearward travel of the inner push rod 251.

The outer push rod 275 is biased towards the rearward part of the actuator by means of the relatively weak compression spring 278 acting between the actuator housing 271 and a spring seat 279 on the outer push rod 275. The rearward travel of the outer push rod 275 is limited by a dead stop 2710 formed in the actuator housing. The actuator is shown with the thumb button slightly depressed (the outer pushrod spring seat 279 is forward of the dead stop 2710). Forward movement of the thumb button 39 and outer push rod 275 pushes the forward end of hollow cylindrical shaft 252 onto the sprung clutch members 63. The biasing spring 272 is stronger than the sprung jaws 63 of the clutch. This prevents the inner shaft 251 from moving forwards until the sprung members 63 have been forced shut around the rear of the lancet piston 111. Continued forward movement of the outer push rod drives the closed clutch and captive lancet piston forward, with the biasing spring 272 ensuring that the clutch remains closed during forward and rearward travel. On release of the thumb button both biasing springs 272 and 278 co-operate to drive the outer push rod 275 and inner push rod 251 back together. When the rearward travel of the inner shaft is arrested by the dead stop 277 the rearward travel of the outer push rod 275 continues to the dead stop 2710 due to the biasing spring 278, thus retracting the hollow cylindrical forward part 252 of the outer push rod 275 off the sprung members 63 and allowing the clutch to open and release the lancet piston with the lancet piston tip securely shielded by the lancet housing.

The lancet housing of the disposable device may comprise three distinct sections of differing internal diameter, with two short transition regions between these sections. The widest (most rearward) section of the lancet housing may be adapted to releasably engage with the actuator body using a snap-fit feature or similar that allows the user to pick up an individual disposable device from a supported array of such devices, oriented vertically with the widest section of the lancet housing uppermost. Holding the actuator in one hand and using a simple stabbing motion, the forward end of the actuator can be inserted into this widest section of lancet housing until a click-fit is felt, whereupon the user can withdraw the actuator with the disposable device attached. At this point there is no secure engagement between the actuator push rod and the grip feature at the rearward end of the lancet piston, as the sprung-open jaws of the actuator push rod are not engaged on the grip feature, and are preferably recessed within the actuator housing to avoid accidental damage to the jaws. The intermediate diameter section of the lancet housing is sized to compress the clutch jaws into engagement with the lancet piston and allow the lancet piston and actuator push rod to move together as a single unit during dispensing of liquid, skin puncture and aspiration. The narrowest, most forward section of bore in the disposable device is sized to make sealing contact with the lancet piston, over part of the bore length (rod seal) or over the entire length of the most forward section of bore (piston seal).

Ejection of the used disposable device is performed by use of an independent ejection mechanism of conventional design, as commonly employed in commercial air displacement pipettors. This mechanism, operated by a second thumb button on the rearward end of the actuator when the lancet piston is in the after-use position, pushes directly on the extreme rearward end of the lancet housing, disengaging the snap-fit or similar connection to the actuator housing and causing axial separation of the disposable device from the actuator. The eject mechanism could be arranged to interlock with the actuator push rod so that it would be impossible to eject the disposable device when the push rod was extended beyond the before use position.

Embodiments of the present invention offer improvements over the prior art for allergy testing. Embodiments of the present invention shield the lancet piston tip before and after use, provides a precise dose of allergen to the skin and gives good control over the lancing step. By contrast, conventional allergy testing makes use of hand-held lancets that are difficult to control with precision. In conventional allergy prick tests the user is required to aim the lancet at a droplet of allergen on the skin. The preferred embodiments herein deliver the allergen and pierce the skin in one continuous operation where delivery of liquid is co-located with lancing. Conventional allergy test lancets present a risk of needlestick injury because they are not shielded before or after use. Furthermore, the practice of using a dropper to place the allergen liquid on the skin does not provide accurate or economical control of the volume. Embodiments of the present invention have been shown to provide a similar response to the conventional test when using only 2µl of solution instead of the usual 40µl. A further advantage is that liquid contained in the bore of the disposable device is expelled onto the skin exactly where the lancet piston tip will subsequently pierce the skin and surrounds the lancet piston tip during penetration. Conventional prick testing also presents a risk of cross-contamination. The droplet of allergen is often placed on the patient's skin in such a way that the dropper is in contact with the droplet on the skin. It is possible for material from the patient's skin (such as bacteria or dead skin cells) to be transferred to the dropper, which is then placed back in to the vial of allergen solution to be used for the next patient. Embodiments of the present invention eliminate this risk through providing a single-use disposable with the option of a single-use pre-packaged dose.

Embodiments of the present invention offer an improvement over prior art prick test devices where the user has to dip them into a solution to pick up and transfer a droplet to the skin (e.g. the Greerpick) because they offer a means of pre-packaging the allergen dose in a sterile or aseptic form, provide an actuator to control the lancing step, automatically shield the lancet tip after use and allow for a disposable device that is smaller than that which can be held and used by hand alone.

When compared with blister-type allergy test devices such as US5099857, embodiments of the present invention offer the advantage that substantially the entire liquid content of the disposable device is delivered to the skin. As can be seen from US5099857, the allergen capsule has a diaphragm above and below the allergen liquid. Piercing through the top layer could cause some liquid to leak out of the top. The bottom layer may partially seal around the lancet, preventing some liquid from contacting the skin. The act of pushing the lancet tip through a membrane may serve to wipe allergen off the lancet tip, precisely the opposite of what is intended.

US6447482 was proposed as an improvement to US5099857 by providing a needle with a small cross-hole in it to carry liquid under the skin by injection. No liquid is delivered to the surface of the skin. Embodiments of the present invention also offer advantages over US6447482 because they deliver a defined volume of liquid to the skin before piercing through this liquid with a lancet in an equivalent manner to the conventional prick test. They thus create an open wound in contact with a reservoir of liquid on the skin surface and have been shown in preliminary tests to give an equivalent response to a conventional prick test that uses twenty times as much liquid. A response identical to that of the existing test is very important to the medical profession because results can be interpreted in the same way. Embodiments of the present invention are also likely to be more economical than the device in US6447482 because the disposable device of the present invention may have fewer parts, would be much smaller, and does not need a spring or membranes in the disposable part.

It is also an advantage to be able to aspirate an allergen solution from a reservoir. Pre-packaged allergens are described in US5099857 and US6447482. The present inventors recognise the convenience of pre-packaged allergens and the potential benefits of maintaining sterility (or at least low contamination) of the allergens. The present invention allows for such pre-packaged allergens, but also allows the user to load the device with allergen solution by simply aspirating a sample from a reservoir at the point of use. Such flexibility is important so that the invention may also be used with the wide range of existing allergens only available in vials. The devices of US5099857 and US6447482 cannot do this. It is further a benefit of the present invention that the forward section of the bore may be pushed through a split septum cap of a vial containing allergen solution to allow aspiration of liquid from the vial without taking the cap off. A further benefit of the split-septum cap is that, in withdrawing the forward section of the bore from the vial, any excess liquid adhering to the outside surface is wiped clean, thus allowing for accurate metering of the amount of liquid picked up and subsequently dispensed.

None of the prior art allergy prick test devices are suitable for taking and transferring blood samples.

Conventional laboratory positive displacement pipettes such as the Gilson Microman series and the Eppendorf 4830 are both capable of aspirating and dispensing liquids, but have no capability for piercing the skin. They consist of a reusable actuator and disposable tips that are picked up, used and ejected by the actuator in one-handed operation. A disadvantage in using these commercial devices with a disposable tip containing a positive displacement piston is that the actuator rod is always driven to the its most extended (most forward) position before the spring-loaded actuator clutch jaws open sufficiently for the positive displacement piston grip feature to be gripped by the clutch jaws on the end of the actuator rod, whereupon the actuator jaws and rod are withdrawn slightly to re-engage the spring loading of the jaws onto the grip feature, and further withdrawn to a pre-set start position by axial spring return. If the disposable bore of a conventional positive displacement pipettor was pre-filled with allergen solution, the solution would be ejected during pickup of the disposable, and therefore not be available for a subsequent prick test to be performed. If this mechanism were to be used with a lancet piston, the tip of the lancet would be exposed at pick-up and eject.

Ejection of conventional positive displacement tips has similar issues, as in most commercial positive displacement pipettors tip ejection is performed by axially pushing the snap-fitted lancet housing out of contact with the actuator housing just after the normally sprung-shut actuator clutch jaws have opened during the last few millimetres of forward stroke. For example, in the Gilson Microman range of pipettors, the force to eject the tip is transferred to the lancet housing of the tip via a dead stop feature on the rear of the piston when the push rod and clutch is fully extended.

European patent application EP1230895 illustrates a problem to be solved by the current invention. This describes a fluid sampler consisting of a fixed member (a lancet shroud containing a capillary for blood sampling) and a movable lancet. Both the fixed member and the lancet are releasably attached to a re-usable actuator. Figure 4 of the specification demonstrates that the actuator has to be locked in the forward position to push-fit the lancet onto the push rod. In this position, the lancet is projecting and presents a high risk of needle stick injury to the user. To remove the lancet the fixed member would need to be pulled off first, allowing the bare lancet to be pulled off (the lancet is not fixed to the fixed member). This presents an even greater risk of needlestick injury because the potential depth of penetration of the lancet is increased. EP1230895 does not incorporate a means of delivering a liquid to the skin or a vessel. If it were adapted to do so by the inclusion of a piston seal, the actuator mechanism would be wholly unsuited to accepting a pre-filled device because the act of fitting the device to the device to the actuator would push the lancet to the forward end of its travel, expelling any pre-loaded fluid.

Embodiments of the present invention ensure secure pick-up of a pre-filled disposable device without altering substantially the existing position of the lancet piston within the bore. Similarly the ejection of said disposable device is performed without leaving an exposed sharp poking out from the forward end of the device.

Embodiments of the present invention also allow for the bore to be filled with liquid and for this liquid to be retained within the bore after ejection of the disposable device from the actuator. This allows for the device to be pre-filled with allergen solution, or to retain a liquid (e.g. blood) sample. If the bore is capped after an aspiration step, as shown in Figure 1, the disposable device may be used to store and transport a liquid (such as allergen) to the point of use, or to transport a sample of blood to a laboratory or test system, without being attached to the actuator. A further requirement is to ensure that a secure engagement is achieved between the actuator and the lancet piston at some point during the forward stroke, so that retraction of the lancet piston is possible. It is preferred that the clutch mechanism be interlocked (as shown in Figure 8) so that it is not possible to retract the push rod without retracting the lancet piston.

Embodiments of the present invention offer an improvement over conventional lancets and lancet actuators in that they may provide one-handed operation and integration of the entire procedure of selecting, unpacking and attaching a disposable lancet in a safe condition (no sharps exposed) to a reusable actuator, using the assembled device to make a puncture wound in the skin, collecting the emergent liquid sample from the skin into the same disposable device, dispensing part or all of the sample and finally ejecting the used disposable in a safe condition (no sharps exposed) for disposal.

By contrast, commercially available lancing systems comprising an insert moulded lancet and a compatible actuator require manual unpackaging and partial disassembly of the actuator to allow attachment, and require the fitting of a safety cap to cover the exposed sharp after removal from the actuator. It is normal practice with conventional lancing systems to uncap and expose the sharp tip of the lancet during manual insertion into the actuator, and then to manually reassemble the actuator forward end over the exposed sharp tip.

Recently, some commercially available integrated systems have overcome this problem by supplying many lancets housed before and after use in separate compartments of an indexable, disposable cartridge that is inserted into a compatible actuator unit. This eliminates the danger of sharps exposure during attachment, but the complexity and cost of these units (per lancet) is high compared to conventional lancing systems that use individual lancets, and integrated systems usually require complex multi-part linkages to coordinate the operations of lancet attachment to actuator, lancet stroke, return stroke and indexing of the cartridge. By contrast, embodiments of the present invention are simpler in design and construction for the reusable actuator and for the disposable parts, and potentially can be made at lower cost Additionally, they can be arranged to aspirate or dispense liquids.

Also recently, the safety lancet market has burgeoned with new competing single-use devices, which are both actuator and lancet, integrated to form a single disposable device. Throwing away the actuator each time with the lancet makes these safety lancets expensive relative to conventional lancets used with a reusable actuator. The present inventors consider that an advantage of the embodiments of the present invention compared to currently available safety lancets is that the cost to the user is potentially lower (per lancet used), there is less waste plastic produced per use and it is possible to collect and transfer the body fluid sample to another location, such as the sample entry point of a conventional lateral flow test device, and to deposit the collected sample before safely ejecting the used lancet device. The embodiments of the present invention can combine the benefit of a compact and cheap disposable lancet with a re-usable actuator with a higher quality lancing mechanism featuring adjustable lancing depth and force. Existing safety lancets have not addressed sample collection, transfer and delivery, and are intended instead to leave a drop of blood on the skin at the puncture site, for pickup and transfer by a second device based on either capillary wicking or forced aspiration.

The embodiments of the present invention offer advantages over prior art devices for taking and/or transferring blood, ISF or liquid tissue samples. They offer a minimal number and size of disposable components (being the lancet piston and bore alone). In contrast, devices such as that of WO2005/094680 are intended to be large enough to be operated directly by hand and consequently consist of a plurality of relatively large parts (the device of WO2005/094680 has five or more parts).

Many of the prior art devices cannot be used on different patients because the part that comes into contact with the patient and their blood is not removable (e.g. US5368047). Of particular advantage in the embodiments of the present invention is the ability to eject both the lancet housing and lancet piston simultaneously as a single ejected assembly in a safe condition without the need to touch either component.

US5569287 offers an improvement over some of the prior art through minimising the disposable component to just a lancet and blood collecting vessel. The embodiments of the present invention offer advantages over this prior art by providing positive displacement aspiration and dispensing, the ability to eject the disposable part in a safe condition, and the ability to define both the aspiration and dispense volumes.

The embodiments of the present invention offer further advantages by providing a means to store and dispense a liquid before lancing and a means to store a sealed blood sample in the disposable device when removed from the actuator. This latter advantage enables a blood sample to be taken with the device, which is then capped and can be transported to another location over an extended period (perhaps days or more) and stored or dispensed thereafter. The blood sample is contained by means of the lancet piston seal and a cap. Moreover, the lancet piston is retained within the bore by friction or through a specific retaining feature such as a bump-off feature, to prevent the lancet piston moving and causing leakage during transport.

Some of the embodiments of an earlier invention by the present inventors and described in PCT/GB/2005/003534 may be combined with the present invention to provide a single-use disposable device and re-usable actuator system with the benefits described herein. This could include stand-off features to hold the forward end of the bore off the skin.

If the bore has an ID of less than 5mm, preferably 0.2mm to 3mm, the surface tension of aqueous liquid (such as blood) will assist in retaining the liquid within the bore and avoid the liquid from dripping out of the forward end when the end of bore it is taken out of a liquid sample. A small diameter aperture to the forward end of the bore is equally important to ensure that air is not drawn past the liquid into the device when aspirating a sample. It is further important to have a relatively small bore ID aperture to the forward end of the bore to enable the end of the bore to be immersed in a droplet of blood on the skin, said droplet being typically of the order of only a few millimetres across. A small aperture is also important where a liquid sample is to be ejected to a small area (for example, a test strip, or small region of the skin). Apertures in the range of 0.2 to 3 mm ID for the end of the bore are preferred because in practise an aperture of up to 3 mm ID is effective at retaining liquids through surface tension and at localising delivered droplets (the smaller the aperture, the better), and the aperture has to be of larger ID than the OD of the piercing end of the lancet piston that has to pass through the aperture. A practical lower limit for the shaft of a lancet to pierce skin is likely to be 0.1 mm OD or above. It should be noted that liquid drawn into the bore will be held in place in the bore both by surface tension and by the fact that the rearward end of the bore is sealed by the lancet piston. In use, liquid acts to seal the very small gap between the bore and piston if such a gap exists. This sealed rearward end of the bore would create a vacuum in the bore forward of the seal to oppose the tendency for dripping of the aspirated sample from the forward end of the bore under the influence of gravity.

From one viewpoint, the embodiments of the invention combine features of lancing and pipetting. Known pipetting devices are intended solely to aspirate and dispense liquids. Known blood sampling devices are intended to acquire a sample and perhaps to integrate such sample acquisition with some form of measurement.

Preferred embodiments of the invention incorporate a lancet tip specifically to pierce the skin, a method and mechanism to drive a lancing operation with associated releasable attachment means, a pipetting function, features to ensure low mass and low friction during the lancing step, and chambers or test-strip chemistry for integrated analysis.

Preferred embodiments of the invention have been described by way of example. Modifications of these embodiments, further embodiments and modifications thereof will be apparent to the skilled person on reading this disclosure and as such are within the scope of the invention.

## Claims

1. A kit of a disposable single use medical device and an actuator device (61), the single use medical device being for delivering liquid to skin before lancing and/or aspirating liquid after lancing, the single use medical device having:
a lancet housing (52) having attachment means (510) for releasable attachment of the lancet housing (52) to the actuator device (61);
a lancet piston (51) having a sharp tip suitable for pricking skin at a forward end and a
gripping feature (57) at a rearward end;
the actuator device (61) having an actuator member, the actuator member having;
lancet piston engagement means comprising a set of normally sprung-open clutch jaws (63);
activation means comprising a cylindrical housing (252) moveable with respect to the clutch jaws (63) so that on movement of the cylindrical housing (252) towards the normally sprung-open jaws (63), the jaws are closed,
wherein the lancet piston engagement means and the activation means are operable to cause releasable engagement between the lancet piston (51) and the actuator member at a first axial position of said lancet piston such that the lancet piston remains fixed in said first axial position relative to the actuator device (61) and the lancet housing (52) until said jaws (63) are closed, and wherein the lancet piston (51) is moveable with respect to the lancet housing (52) axially along an internal space of the lancet housing, the lancet piston slidably and sealingly fitting in at least one section of the bore (53) of the lancet housing (52), the device having a before-use configuration in which the sharp tip of the lancet piston (51) is held shielded by the lancet housing (52), the releasable engagement between the actuator member and the lancet piston (51) being operable to allow movement of the lancet piston (51) to expose the sharp tip forwardly from the lancet housing (52) to prick the skin and to move the sharp tip of the lancet piston (51) to an after-use configuration in which the sharp tip of the lancet piston is again held shielded by the lancet housing (52), there being a liquid-containing space in the bore (53) forwardly of the lancet piston in either the before-use configuration or in the after-use configuration or both.

2. A kit according to claim 1 wherein the activation means is operable to cause release of the engagement between the lancet piston (51) and the actuator member at a second axial position of the lancet piston (51), the sharp tip of the lancet piston (51) being shielded by the lancet housing (52) in said second position.

3. A kit according to claim 1 or claim 2 wherein a section (56) of the lancet housing (52) forward of the first axial position acts as an interlock to constrain and prevent release of the engagement between the lancet piston (51) and actuator member at a position forward of the before-use and/or after-use location of the lancet piston.

4. A kit according to any one of claims 1 to 3 having at least one stop element for urging against accidental exposure of the sharp tip of the lancet piston (51) from the lancet housing (52) before or after the lancet piston (51) is engaged with the actuator member.

5. A kit according to any one of claims 1 to 4 wherein the lancet piston (51) and lancet housing (52) co-operate to hold the lancet piston (5 1) in a fixed axial position relative to the lancet housing (52), such co-operation being capable of being overcome by operation of the actuator in use.

6. A kit according to any one of claims 1 to 5 including at least one locator lancet piston guide element (55) for maintaining the axial and/or radial position of the lancet piston (51) with respect to the lancet housing (52) before and/or after engagement with the actuator member.

7. A kit according to any one of claims 1 to 6 wherein seal means is provided, operable substantially to prevent flow of liquid from the liquid-containing space and substantially to prevent air flow into the liquid-containing space past the seal means on movement of the lancet piston (51) or liquid from the liquid-containing space past the seal means on movement of the lancet piston, the seal means being in sliding engagement with a sealing surface, one of the seal means and the sealing surface being fixedly movable with the lancet piston at least during forward displacement of the lancet piston, so that displacement of the lancet piston either from the before-use configuration to the exposed position or from the exposed position to the after-use configuration provides at least one of:
(i) suction for drawing liquid into and along the liquid-containing space from a forward end of the bore (53), and
(ii) pressure for expelling liquid from the liquid-containing space via a forward end of the bore (53).

8. A kit according to any one of claims 1 to 7 including a plurality of said disposable medical devices.

9. A method of operating a kit according to any one of claims 1 to 8, including the steps:
releasably attaching the actuator device (61) to the lancet housing (52) of the disposable medical device;
releasab ly engaging the actuator member and the lancet piston (51) via said activation means.

10. A method according to claim 9 further including the steps of:
releasing engagement between the lancet piston (51) and actuator member, optionally via said activation means at the after-use position; and
removing or ejecting the disposable medical device from the actuator (61), so that the lancet piston (51) tip remains shielded by the lancet housing (52).

## Patentansprüche

1. Set aus einer medizinischen Wegwerfvorrichtung zur einmaligen Verwendung und einer Aktuatorvorrichtung (61), wobei die medizinische Vorrichtung zur einmaligen Verwendung dazu dient, der Haut vor dem Einstechen Flüssigkeit zuzuführen und/oder nach dem Einstechen Flüssigkeit abzusaugen, wobei die medizinische Vorrichtung zur einmaligen Verwendung Folgendes aufweist:
ein Lanzettengehäuse (52) mit Haftmitteln (510) zur lösbaren Befestigung des Lanzettengehäuses (52) an die Aktuatorvorrichtung (61);
einen Lanzettenkolben (51) mit einer scharfen Spitze, die zum Einstechen von Haut geeignet ist, am vorderen Ende und einem Greifelement (57) am hinteren Ende;
wobei die Aktuatorvorrichtung (61) ein Aktuatorelement aufweist, wobei das Aktuatorelement Folgendes aufweist:
ein Lanzettenkolbeneingriffsmittel, das einen Satz im Normalfall aufgespreizter Kupplungsklauen (63) umfasst;
ein Aktivierungsmittel, das ein zylindrisches Gehäuse (65) umfasst, welches in Bezug auf die Kupplungsklauen (63) bewegbar ist, so dass bei Bewegung des zylindrischen Gehäuses (65) in Richtung der im Normalfall aufgespreizten Kupplungsklauen (63) die Klauen geschlossen werden,
worin das Lanzettenkolbeneingriffsmittel und das Aktivierungsmittel so betätigbar sind, dass sie einen lösbaren Eingriff zwischen dem Lanzettenkolben (51) und dem Aktuatorelement in einer ersten axialen Position des Lanzettenkolbens in einer Weise herbeiführen, dass der Lanzettenkolben in der ersten axialen Position relativ zu der Aktuatorvorrichtung (61) und dem Lanzettengehäuse (52) fixiert bleibt, bis die Klauen (63) geschlossen sind, und worin der Lanzettenkolben (51) in Bezug auf das Lanzettengehäuse (52) axial entlang eines Innenraums des Lanzettengehäuses bewegbar ist, wobei der Lanzettenkolben gleitbar und dichtend in zumindest einen Abschnitt des Bohrlochs (53) des Lanzettengehäuses (52) passt, die Vorrichtung eine Konfiguration vor Gebrauch aufweist, bei der die scharfe Spitze des Lanzettenkolbens (51) durch das Lanzettengehäuse (52) abgeschirmt bleibt und der lösbare Eingriff zwischen dem Aktuatorelement und dem Lanzettenkolben (51) so betätigbar ist, dass dies eine Bewegung des Lanzettenkolbens (51) von dem Lanzettengehäuse (52) weg nach vorne ermöglicht, die die scharfe Spitze freilegt, um die Haut einzustechen und die scharfe Spitze des Lanzettenkolbens (51) in eine Konfiguration nach Gebrauch zu bewegen, bei der die scharfe Spitze des Lanzettenkolbens wieder durch das Lanzettengehäuse (52) abgeschirmt wird, wobei es entweder in der Konfiguration vor Gebrauch oder in der Konfiguration nach Gebrauch oder in beiden Konfigurationen einen Flüssigkeit fassenden Bereich im Bohrloch (53) gibt, der sich weiter vorne als der Lanzettenkolben befindet.

2. Set nach Anspruch 1, worin das Aktivierungsmittel so betätigbar ist, dass es das Lösen des Eingriffs zwischen dem Lanzettenkolben (51) und dem Aktuatorelement in einer zweiten axialen Position des Lanzettenkolbens (51) verursacht, wobei die scharfe Spitze des Lanzettenkolbens (51) in der zweiten Position von dem Lanzettengehäuse (52) abgeschirmt wird.

3. Set nach Anspruch 1 oder Anspruch 2, worin ein Abschnitt (56) des Lanzettengehäuses (52), der weiter vorne als die erste axiale Position liegt, als Sperre fungiert, um das Lösen des Eingriffs zwischen dem Lanzettenkolben (51) und dem Aktuatorelement in einer Position, die weiter vorne liegt als die Position des Lanzettenkolbens vor Gebrauch und/oder jene nach Gebrauch, zu behindern und zu unterbinden.

4. Set nach einem der Ansprüche 1 bis 3 mit zumindest einem Anschlagelement zum Vortrieb gegen die unbeabsichtigte Bloßlegung der scharfen Spitze des Lanzettenkolbens (51) von dem Lanzettengehäuse (52), bevor oder nachdem der Lanzettenkolben (51) mit dem Aktuatorelement im Eingriff steht.

5. Set nach einem der Ansprüche 1 bis 4, worin der Lanzettenkolben (51) und das Lanzettengehäuse (52) darin zusammenwirken, den Lanzettenkolben (51) in einer fixierten axialen Position relativ zu dem Lanzettengehäuse (52) zu halten, wobei dieses Zusammenwirken durch die Betätigung des Aktuators in Verwendung überwunden werden kann.

6. Set nach einem der Ansprüche 1 bis 5, das zumindest ein Sucherlanzettenkolbenführelement (55) zur Beibehaltung der axialen und/oder radialen Position des Lanzettenkolbens (51) in Bezug auf das Lanzettengehäuse (52) vor und/oder nach dem Eingriff mit dem Aktuatorelement umfasst.

7. Set nach einem der Ansprüche 1 bis 6, worin eine Dichtungsvorrichtung bereitgestellt ist, das so betätigbar ist, dass es im Wesentlichen den Ausfluss der Flüssigkeit aus dem Flüssigkeit fassenden Bereich verhindert und im Wesentlichen einen Luftstrom in den Flüssigkeit fassenden Bereich durch die Dichtungsvorrichtung hindurch bei Bewegung des Lanzettenkolbens (51) oder jener von Flüssigkeit aus dem Flüssigkeit fassenden Bereich durch die Dichtungsvorrichtung hindurch bei Bewegung des Lanzettenkolbens verhindert, wobei die Dichtungsvorrichtung in gleitendem Eingriff mit einer Dichtungsoberfläche steht und entweder die Dichtungsvorrichtung oder die Dichtungsoberfläche zumindest während des Versetzung des Lanzettenkolbens nach vorne mit dem Lanzettenkolben fixiert bewegbar ist, so dass die Versetzung des Lanzettenkolbens entweder aus der Konfiguration vor Gebrauch in die freigelegte Position oder von der freigelegten Position in die Konfiguration nach Gebrauch zumindest eine der beiden folgenden Wirkungen nach sich zieht:
(i) Saugwirkung zum Ziehen der Flüssigkeit in den und entlang dem Flüssigkeit fassenden Bereich aus einem vorderen Ende des Bohrlochs (53), und
(ii) Druck zum Pressen der Flüssigkeit aus dem Flüssigkeit fassenden Bereich durch ein vorderes Ende des Bohrlochs (53).

8. Set nach einem der Ansprüche 1 bis 7, das eine Vielzahl der medizinischen Wegwerfvorrichtungen umfasst.

9. Verfahren zur Anwendung eines Sets nach einem der Ansprüche 1 bis 8, das die folgenden Schritte umfasst:
das lösbare Befestigen der Aktuatorvorrichtung (61) an dem Lanzettengehäuse (52) der medizinischen Wegwerfvorrichtung;
das Herstellen eines lösbaren Eingriffs zwischen dem Aktuatorelement und dem Lanzettenkolben (51) mittels des Aktivierungsmittels.

10. Verfahren nach Anspruch 9, das weiters die folgenden Schritte umfasst:
das Lösen des Eingriffs zwischen dem Lanzettenkolben (51) und dem Aktuatorelement, gegebenenfalls mittels des Aktivierungsmittels in der Position nach Gebrauch; und
das Entfernen oder Auswerfen der medizinischen Wegwerfvorrichtung aus dem Aktuator (61), so dass die Spitze des Lanzettenkolbens (51) von dem Lanzettengehäuse (52) abgeschirmt bleibt.

## Revendications

1. Kit d'un dispositif médical jetable à usage unique et d'un dispositif d'actionneur (61), le dispositif médical à usage unique étant pour la délivrance de liquide à la peau avant le lancement et/ou l'aspiration du liquide après le lancement, le dispositif médical à usage unique comportant:
un boîtier de lancette (52) ayant un moyen de fixation (510) pour la fixation amovible du boîtier de lancette (52) au dispositif actionneur (61);
un piston de lancette (51) ayant une pointe affûtée apte à piquer la peau à une extrémité avant et une caractéristique de préhension (57) à une extrémité arrière;
le dispositif actionneur (61) comportant un élément actionneur, l'élément actionneur comportant:
des moyens de mise en prise de piston de lancette comprenant un ensemble de mâchoires d'embrayage normalement ouvertes (63);
des moyens d'activation comprenant un boîtier cylindrique (65) déplaçable par rapport aux mâchoires d'embrayage (63) de sorte que lors d'un déplacement du boîtier cylindrique (65) vers les mâchoires normalement ouvertes (63), les mâchoires sont fermées,
où les moyens de mise en prise avec le piston de lancette et les moyens d'activation sont actionnables pour provoquer une mise en prise relâchable entre le piston de lancette (51) et l'élément actionneur à une première position axiale dudit piston de lancette de sorte que le piston de lancette reste fixé dans ladite première position axiale relativement au dispositif actionneur (61) et au boîtier de lancette (52) jusqu'à ce que lesdites mâchoires (63) soient fermées, et où le piston de lancette (51) est déplaçable par rapport au boîtier de lancette (52) axialement le long d'un espace interne du boîtier de lancette, le piston de lancette s'adaptant d'une manière coulissante et étanche dans au moins une section du perçage (53) du boîtier de lancette (52), le dispositif ayant une configuration avant utilisation dans laquelle la pointe affûtée du piston de lancette (51) est protégée par le boîtier de lancette (52), la mise en prise relâchable entre l'élément actionneur et le piston de lancette (51) étant actionnable pour permettre le déplacement du piston de lancette (51) afin d'exposer la pointe tranchante vers l'avant depuis le boîtier de lancette (52) pour piquer dans la peau et pour déplacer la pointe tranchante du piston de lancette (51) vers une configuration après utilisation dans laquelle la pointe tranchante du piston de lancette est à nouveau protégée par le boîtier de lancette (52), un espace contenant du liquide étant prévu dans le perçage (53) en amont du piston de lancette soit dans la configuration avant utilisation soit dans la configuration après utilisation ou les deux.

2. Kit selon la revendication 1, dans lequel le moyen d'activation est actionnable pour provoquer la sortie de prise entre le piston de lancette (51) et l'élément actionneur à une deuxième position axiale du piston de lancette (51), la pointe tranchante du piston de lancette (51) étant protégée par le boîtier de lancette (52) dans ladite seconde position.

3. Kit selon la revendication 1 ou la revendication 2, dans lequel une section (56) du boîtier de lancette (52) en amont de la première position axiale agit comme un interverrouillage pour contraindre et prévenir la sortie de prise entre le piston de lancette (51) et l'élément actionneur à une position en amont de l'emplacement avant utilisation et/ou après utilisation du piston de lancette.

4. Kit selon l'une quelconque des revendications 1 à 3 ayant au moins un élément d'arrêt pour solliciter contre une exposition accidentelle de la pointe tranchante du piston de lancette (51) du boîtier de lancette (52) avant ou après que le piston de lancette (51) a été mis en prise avec l'élément actionneur.

5. Kit selon l'une quelconque des revendications 1 à 4, dans lequel le piston de lancette (51) et le boîtier de lancette (52) coopèrent pour tenir le piston de lancette (51) dans une position axiale fixe relativement au boîtier de lancette (52), une telle coopération étant apte à être surmontée par l'opération de l'actionneur en cours d'utilisation.

6. Kit selon l'une quelconque des revendications 1 à 5, comportant au moins un élément de guidage de piston de lancette de localisation (55) pour maintenir la position axiale et/ou radiale du piston de lancette (51) relativement au boîtier de lancette (52) avant et/ou après la mise en prise avec l'élément actionneur.

7. Kit selon l'une quelconque des revendications 1 à 6, dans lequel un moyen d'étanchéité est réalisé, actionnable sensiblement pour empêcher l'écoulement du liquide de l'espace contenant du liquide et pour empêcher sensiblement un écoulement d'air dans l'espace contenant du liquide devant le moyen d'étanchéité lors d'un déplacement du piston de lancette (51) ou du liquide de l'espace contenant du liquide devant le moyen d'étanchéité lors du déplacement du piston de lancette, le moyen d'étanchéité étant en prise de coulissement avec une surface d'étanchéité, un parmi le moyen d'étanchéité et la surface d'étanchéité étant déplaçable fixement avec le piston de lancette au moins durant un déplacement vers l'avant du piston de lancette de sorte que les déplacements du piston de lancette soit de la configuration avant utilisation à la position exposée soit de la position exposée à la configuration après utilisation réalise au moins une parmi:
(i) aspiration pour aspirer le liquide dans et le long de l'espace contenant du liquide d'une extrémité avant du perçage (53), et
(ii) pression pour expulser le liquide de l'espace contenant du liquide par une extrémité avant du perçage (53).

8. Kit selon l'une quelconque des revendications 1 à 7, comportant plusieurs desdits dispositifs médicaux jetables.

9. Procédé d'exploitation d'un kit selon l'une quelconque des revendications 1 à 8, comportant les étapes:
fixer amoviblement le dispositif actionneur (61) au boîtier de lancette (52) du dispositif médical jetable;
mettre en prise relâchablement l'élément actionneur et le piston de lancette (51) par ledit moyen d'activation.

10. Procédé selon la revendication 9, comportant en outre les étapes de:
libérer la mise en prise entre le piston de lancette (51) et l'élément actionneur, optionnellement par ledit moyen d'activation à la position après utilisation; et
retirer ou éjecter le dispositif médical jetable de l'actionneur (61) de sorte que la pointe du piston de lancette (51) reste protégée par le boîtier de lancette (52).
